# EUROPEAN PATENT SPECIFICATION

(11) **EP 4 293 079 B1**
(45) Date of publication and mention of the grant of the patent: **16.04.2025**
(21) Application number: 22204222.8
(22) Date of filing: 27.10.2022
(51) Int. Cl.: C08L 67/02, C08L 67/04, C08K 3/22, C08K 3/26, C08K 5/20, C08L 3/02, C08L 23/04, C08L 23/08

(54) **BIODEGRADABLE POLYMER COMPOSITION**
BIOLOGISCH ABBAUBARE POLYMERZUSAMMENSETZUNG
COMPOSITION POLYMÈRE BIODÉGRADABLE

(43) Date of publication of application: 20.12.2023
(73) Proprietor: BASF SE, 67056 Ludwigshafen (DE)
(72) Inventor: LOOS, Robert, 67056 Ludwigshafen am Rhein (DE); LOHMANN, Jerome, 67056 Ludwigshafen am Rhein (DE); EFFEN, Norbert, 67056 Ludwigshafen am Rhein (DE); AUFFERMANN, Joerg, 67056 Ludwigshafen am Rhein (DE)
(74) Representative: BASF IP Association

(56) References cited:
- EP-A1- 2 679 633
- FR-A1- 3 028 520
- US-A- 6 005 068
- US-A1- 2009 324 917

## Description

The present invention relates to a biodegradable polymer composition containing at least one poly(meso-lactide) and at least one biodegradable polyester selected from aliphatic polyesters, aliphatic-aromatic polyesters and copolymers and mixtures thereof; films comprising the biodegradable polymer composition and packaging and mulch films comprising such films.

Compositions comprising biodegradable polyesters like polybutylene-adipate-terephthalate are widely used in the manufacture of flexible films for different applications, e.g. mulch films, consumer bags and waste bags. The addition of the rigid polymer polylactide ("PLA") to the biodegradable polyesters increases the E-modulus and tensile strength of the polyester composition and also improves the processability of the composition during film blowing.

Thin films made from the biodegradable composition with a thickness of roughly 6 to 100 micron are usually manufactured by film extrusion or blown film extrusion. Therefore, these films usually show an anisotropy between the transport direction of the machine ("machine direction") and the direction perpendicular to the transport direction of the machine ("transversal direction"). This anisotropy results inter alia in different values of the E-modulus, the tensile strength, the elongation at maximum tensile strength, and the tear resistance. The latter one is particular important for the manufacture of thin bags and especially if the bags possess a carrier handle. For the stability of the bags and especially the carrier handle the tear resistance should be sufficiently high, otherwise the bag and especially the carrier handle is not stable and may already tear at low weights of the goods carried in the bag. This is e.g. induced by small movements up and down of the bag and/or small holes teared into the bag by goods having sharp edges.

Further interesting properties of films used for packaging like bags for fruits and vegetables are the optical characteristics, i.e. the total transmittance, haze and clarity of the films. Due to the immiscibility of the PLA and biodegradable aliphatic and aliphatic-aromatic polyesters present in the composition, the composition has a multiphase structure which may lead to undesirable non-transparent films.

For mulch films the elongation at maximum tensile strength in machine direction is an important parameter since mulch films are applied onto the field in machine direction. A higher elongation at maximum tensile strength in the machine direction results in a higher energy absorption by the film during the laying down process on the field by e.g. a tractor and hence allows a faster speed and less unwanted tearing of the films for the farmer. The addition of PLA to the biodegradable aliphatic and aliphatic-aromatic polyesters inevitably leads to a significant decrease of the elongation at maximum tensile strength especially at higher PLA contents.

In view of the general efforts for reducing the consumption of mineral oil based resources, higher amounts of bio-based PLA and other bio-based raw materials in the biodegradable compositions are desirable. Unfortunately, further increase of the amount of PLA usually has undesirable side effects on the properties of the films produced. The sum of the important tear resistance values in machine and transversal direction of the films are reduced with increasing amounts of the very rigid PLA. Furthermore, the values of the tear resistance in transversal direction and machine direction of the films change their order, i.e. at low PLA contents the tear resistance in transversal direction is larger than the tear resistance in machine direction, but with increasing amounts of PLA the tear resistance in transversal direction becomes lower than the tear resistance in machine direction. Additionally, the elongation at maximum tensile strength also decreases with increasing PLA content.

EP 2074175B1 describes films made of compositions containing a continuous phase of thermoplastic polymer incompatible with starch, e.g. biodegradable aliphatic-aromatic polyesters, polyhydroxyalkanoates, polyethers and polyamides, and a disperse phase made of destructurized starch, and a further dispersed phase of a rigid polymer such as polylactide acid and polyglycolic acid. The films show a substantial isotropy of the two longitudinal and transverse direction in relation to tear propagation and no propagation of any lateral fractures.

EP 3070111B1 relates to compositions for use as films comprising a polymer of natural origin, e.g. starch, and a biodegradable aliphatic-aromatic copolyester containing aliphatic long chain and short chain diacids. The composition may further comprise a rigid polymer, e.g. a polyhydroxyalkanoate like polylactic acid and polyglycolic acid. The film shown in the experimental section has a lower tear resistance in transversal direction than in machine direction.

EP 2781551A1 describes biodegradable resin composition and a biodegradable film made thereof. The composition comprises at least 4 components, namely starch, an aliphatic polyester, a non-crystalline polylactide acid and an aliphatic-aromatic polyester. The use of non-crystalline PLA in the compositions leads to improved film properties compared to film prepared from compositions containing crystalline PLA.

US 6 005 068 A discloses in example 1 a film made from a polymer blend comprising 38.6 wt% polycaprolactone, and 61.15 wt% polylactide having a number-average molecular weight of 157,900 and a 10% meso-lactide content, and 0.25 wt% diatomaceous earth.

An object of the present invention was to provide biodegradable polymer compositions comprising PLA and biodegradable polyester with increased content of bio-based components which are suited for the manufacture of flexible films with improved mechanical properties, in particular for the manufacture of thin flexible films wherein the tear resistance in transversal direction is sufficiently high and having improved elongation at maximum tensile strength in machine direction. Preferably, the films prepared from such biodegradable polymer compositions have improved optical properties with respect to transmittance, haze and clarity.

These objects could be achieved by the following biodegradable polymer composition containing
a1) 2 to 50 wt.-%, based on the total weight of components a1) to e) of the polymer composition, of at least one one poly(meso-lactide) having a number-average molecular weight of at least 20 000 g/mol and a ratio of L-lactic units : D-lactic units of at least 20:80 up to 80:20;
a2) 0 to 48 wt.-%, based on the total weight of components a1) to e) of the polymer composition, of at least one polylactide a2) having a ratio of L-lactic units : D-lactic units of above 65:35 or below 35:65 and wherein the sum of the weight percentages of poly(meso-lactide) a1) and polylactide a2) is at maximum 50 wt.-%, based on the total weight of components a1) to e);
b) 20 to 98 wt.-%, based on the total weight of components a1) to e) of the polymer composition, of at least one biodegradable polyester selected from aliphatic polyesters, aliphatic-aromatic polyesters and copolymers and mixtures thereof;
c) 0 to 55 wt.-%, based on the total weight of components a1) to e) of the polymer composition, of at least one starch polymer;
d) 0 to 40 wt.-%, based on the total weight of components a1) to e) of the polymer composition, of at least one inorganic filler; and
e) 0 to 5 wt%, based on the total weight of components a1) to e) of the polymer composition, of at least one compound selected from cross-linking agents, lubricants, chain extenders, stabilizers, nucleating agents, slip and release agents, surfactants, waxes, antistatic agents, antifoggants, dyes, pigments, UV absorbers, UV stabilizers and other plastics additives.

The present invention also relates to films comprising the biodegradable polymer composition and to packaging like carrier bags, and fruit and vegetable bags; waste bags, and mulch films comprising the films according to the present invention.

Surprisingly it was found that biodegradable compositions containing poly(meso-lactide) as PLA component yield biodegradable films with improved mechanical and/or optical properties.

In the following the invention will be described in detail.

### Poly(meso-lactide) a1):

The polymer composition contains as component a1) one or more poly(meso-lactide).

The term "poly(meso-lactide)" as used herein means a polymer or copolymer containing mainly repeating units derived from meso-lactide. It is also abbreviated as PMLA herein. Meso-lactide is the cyclic diester of a D-lactic acid and a L-lactic acid. Essentially, the homopolymerization of meso-lactide yields a polymer wherein the D-lactic acid units and the L-lactic acid units are distributed quite regular in the polymer chain, since there could be a reaction by two L-lactic acids units or two D-lactic acids units (head to head reaction) resulting in the sequence -(L-D-D-L-)ₙ or -(D-L-L-D-)ₙ or there can be a reaction of the L-lactic acid unit with a D-lactic acid unit (head to tail reaction) resulting in the sequence -(D-L-D-L)ₙ. This means the average sequence length, also called average block length of L-lactc units and D-lactic units resulting from the ring opening polymerization and neglecting any transesterification reactions is at a minimum 1 and at a maximum 2. Random polymerization of meso-lactide yields an average sequence length of the consecutive D- and L-lactic acid units between these limits, i.e. between 1 and 2. Additionally, the ratio of D- and L-lactic acid units in the polymers derived from meso-lactide only is close to 1:1.

The term "units derived from lactic acids", also referred to as "lactic units", means the monomeric lactic acid units derived from L-lactide acid or D-lactic acid.

The term "rac-lactide" means the high-melting material known as racemic-lactide which is formed by melting a mixture of about 50% L-lactide and 50% D-lactide.

The poly(meso-lactide) a1) used in the biodegradable polymer composition has a ratio of L-lactic units : D-lactic units of at least 20:80 up to 80:20, preferable of at least 25:75 up to 75:25, more preferred of at least 30:70 up to 70:30, even more preferred the ratio of L-lactic units : D-lactic units is at least 35:65 up to 65:35 and in particular preferred the ratio is at least 40:60 up to 60:40.

The poly(meso-lactide) a1) may be a homopolymer of meso-lactide or a copolymer of at least 40% meso-lactide and up to 60% of another lactide, preferably of at least 50% meso-lactide and up to 50% of another lactide, more preferred at least 60% meso-lactide and up to 40% of another lactide, even more preferred at least 70% meso-lactide and up to 30% of another lactide, and in particular preferred at least 80% meso-lactide and up to 20% of another lactide. Within these ranges the poly(meso-lactide) a1) is preferably a homopolymer of meso-lactide or a copolymer of at least 88% meso-lactide and up to 12% of another lactide, and in particular preferred of at least 90% meso-lactide and up to 10% of another lactide. If it is a copolymer, then the copolymer may be a random and/or block copolymer. The other lactide may be any other lactide including L-lactide, D- lactide, or rac-lactide, or a mixture of any two or more thereof.

The poly(meso-lactide) a1) may further contain repeating units formed from other monomers that are co-polymerizable with meso-lactide or D- or L-lactide, such as alkylene oxides (including ethylene oxide, propylene oxide, butylene oxide, tetramethylene oxide, and the like), cyclic lactones, or carbonates. Repeating units derived from these other monomers can be present in block and/or random arrangements. These other repeating units may constitute up to 10% by weight of the poly(meso-lactide), preferably from 0% to 5% by weight, especially preferred from about 0% to 2% by weight, of the PMLA, and may be absent.

According to the invention at least 90%, preferably at least 95% of the weight of the PMLA used as component a1) is made up of units derived from lactic acids, wherein the ratio of L-lactic units : D-lactic units is at least 20:80 up to 80:20, preferable is at least 25:75 up to 75:25, more preferred is at least 30:70 up to 70:30, even more preferred the ratio of L-lactic units : D-lactic units is at least 35:65 up to 65:35 and in particular preferred the ratio is at least 40:60 up to 60:40.

Preferably at least 90%, more preferred at least 95% of the weight of the PMLA used as component a1) is made up of lactic units, wherein at least 80% of the lactic units are formed by polymerizing meso-lactide preferably at least 88% of the lactic units are formed by polymerizing meso-lactide , and more preferred at least 90% of the lactide units are formed by polymerizing meso-lactide.

The PMLA may also contain residues of an initiator compound, which is often used during the polymerization process to provide molecular weight control. Suitable such initiators include, for example, water, alcohols, polyhydroxy compounds of various types (such as ethylene glycol, propylene glycol, polyethylene glycol, polypropylene glycol, other glycol ethers, glycerin, trimethylolpropane, pentaerythritol, hydroxyl-terminated butadiene polymers, and the like), polycar-boxyl- containing compounds, and compounds having at least one carboxyl and one hydroxyl group (such a lactic acid or lactic acid oligomer). The initiator residue preferably constitutes no more than 5%, and especially no more than 2% of the weight of the PMLA, except in the case in which the initiator is a residue of a lactic acid or lactic acid oligomer, which can constitute any proportion of the PMLA.

The PMLA may have long-chain branches (having 3 or more carbon atoms). Long-chain branches can be introduced in the polylactide in various ways, such as by reacting carboxyl groups on the polylactide with epoxide groups that are present on an acrylate polymer or copolymer. The acrylate polymer or copolymer is characterized in being a solid at 23°C, containing an average of from about 2 to about 15 free epoxide groups/molecule (such as from about 3 to about 10 or from about 4 to about 8 free epoxide groups/molecule), and being a polymerization product of at least one epoxy-functional acrylate or methacrylate monomer, preferably copolymerized with at least one additional monomer. The acrylate polymer or copolymer suitably has a number-average molecular weight per epoxide group of about 150 to about 700, such as from 200 to 500 or from 200 to 400 g/mol. The acrylate polymer or copolymer suitably has a number-average molecular weight of from 1000 to 6000, such as from about 1500 to 5000 or from about 1800 to 3000 g/mol. Other approaches to introducing long-chain branching are described in U. S. Patent Nos. 5,359,026 and 7,015,302, and in WO 06/002372A2.

In preferred embodiments, the PMLA lacks long-chain branches.

The poly(meso-lactide) has a number-average molecular weight of at least 20 000 g/mol, preferably at least 30 000 g/mol, more preferred at least 50 000 g/mol as measured by GPC in THF against a narrow polystyrene standard. Preferably the poly(meso-lactide) has a number-average molecular weight in the range of 20 000 g/mol to 200 000, more preferred the number-average molecular weights is in the range of 30 000 to 130 000 g/mol, even more preferred 50 000 to 130 000 g/mol.

The poly(meso-lactide) may have a relative viscosity of 1.1 to 6, 1.25 to 5, or 1.5 to 3.5, measured using a 1% wt/vol solution of the poly(meso-lactide) in chloroform against a chloroform standard on a capillary viscometer at 30°C.

The average block length of L-lactic units and of D-lactic units in the poly(meso-lactide) are usually at least 1.0, preferably at least 1.1, more preferred at least 1.2, even more preferred at least 1.25, and most preferred at least 1.3. The upper limit of the average block length of L-lactic units and of D-lactic units in the poly(meso-lactide) is usually up to 2.0, preferably up to 1.75, more preferred up to 1.5, and most preferred up to 1.4. Usually, the average block length is in the rage of 1.0 to 2.0, a preferred range of the average block length of L-lactic units and of D-lactic units is at least 1.1 to 1.75. The average block length can be determined by proton NMR using methods to determine Pm as described by Coates et al., in J. American Chemical Society 2002, 124, 1316, and the following relationship: Average block length = 1+Pm /(1+(1-Pm))
A particular preferred poly(meso-lactide) to be used as poly(meso-lactide) a1) in the biodegradable polymer composition is a poly(meso-lactide) having a number-average molecular weight of at least 20 000 g/mol up to 200 000 g/mol, preferably of at least 30 000 to 130 000 g/mol which contains at least 90 %, preferably at least 95 % of the weight of the PMLA, units derived from lactic acids, wherein ratio of L-lactic units : D-lactic units is in the range of from at least 35:65 up to 65:35, particular preferred the ratio is in the range of at least 40:60 up to 60: 40, and wherein the poly(meso-lactide) has an average block length of at least 1.0 up to 2.0, preferably of at least 1.1 up to 1.75.

Preferably the poly(meso-lactide) has a glass transition temperature of 38°C to 46°C measured by DSC at a relative humidity of 100 % and a heating rate of 20 °C/min of a 5 mg sample after conditioning the sample by melting at 180 °C followed by a quench to erase heat history and to ensure contact with the pan.

The poly(meso-lactide) is characterized as being an amorphous PLA grade. By an "amorphous grade", it is meant that the PMLA contains no more than 5 J/g of crystallites after being heated at 110°C in air for one hour. The sample is previously heated to at least 220°C to melt any crystallites and then quenched by rapidly cooling to room temperature (23 ± 3°C). The quenched sample is then heated at 110°C for one hour and again quenched by cooling to room temperature. Crystallinity then is conveniently measured using differential scanning calorimetry (DSC) methods. The amount of such crystallinity is expressed herein in terms of J/g, i.e., the enthalpy of melting, in Joules, of the polylactide crystals in the sample, divided by the weight in grams of polylactide(s) in the sample. A convenient test protocol for making DSC measurements is to heat a 5-10 milligram sample from 25°C to 225°C at 20°C/minute under air, on a Mettler Toledo DSC 3+ calorimeter running STARe V.16 software, or equivalent apparatus.

The poly(meso-lactide) is produced by polymerizing meso-lactide by itself or by copolymerizing a meso-lactide and another lactide in random and/or block fashion. The polymerization can be conducted batch-wise, semi-continuously, or continuously.

The poly(meso-lactide) may contain residual lactide, which is usually formed as side-product during the manufacture of the polylactide. If present, lactide may constitute up to 20%, up to 15%, up to 10%, up to 5% or up to 2% of the weight of the poly(meso-lactide).

Further details and information about the preparation of the poly(meso-lactide) to be used as component a1) are described in US 5,142,023 and WO 2020/251745A1.

The biodegradable polymer composition comprises 2 to 50 wt.-%, based on the total weight of components a1) to e) of the polymer composition, of the poly(meso-lactide) a1). Preferably the biodegradable polymer composition comprises at least 4 wt.-%, more preferred at least 5 wt.-%, even more preferred at least 7 wt.-%, even more preferred at least 9 wt.-%, most preferred at least 13 wt.-%, and in particular preferred at least 16 wt.-% of the poly(meso-lactide) a1), based on the total weight of components a1) to e) of the biodegradable polymer composition. The maximum concentration of the poly(meso-lactide) a1) in the biodegradable polymer composition is preferably 40 wt.-%, more preferred 30 wt.-%, even more preferred 25 wt.-%, most preferred 20 wt.-% and in particular preferred 18 wt.-%, based on the total weight of components a1) to e) of the biodegradable polymer composition. Preferred ranges of the poly(meso-lactide) a1) in the biodegradable polymer composition are 4 to 40 wt.-%, more preferred 5 to 30 wt.-% and even more preferred 7 to 25 wt.-%, based on the total weight of components a1) to e) of the biodegradable polymer composition.

### Polylactide a2)

The biodegradable polymer composition may contain one or more additional polylactides a2) .

The terms "PLA" and "polylactide" as used herein interchangeably mean polylactic acid, which is obtainable by polymerizing of either D-lactide, L-lactide, meso-lactide or mixtures thereof. In case only D- or only L-lactide are polymerized, the resulting polymer chains consist essentially of D- or L-lactic acid units, respectively. In case of polymerizing of a mixture of D- and L-lactide longer sequences of -(D)ₙ and -(L) ₙ are obtained due to the random polymerization of D- and L-lactide. In case the PLA is prepared from D-lactide and L-lactide only, i.e. without mesolac-tide, the minimum block length of the D- and L-lactic units in the polylactide is 2 from a theoretical point of view. This would only be the case in a strict alternating reaction of D- and L-lactide. The latter also holds true if mixtures of either L-lactide and a minor amount of meso-lactide or D-lactide with a minor amount of meso-lactide are polymerized. Therefore, the average sequence length of D- or L-lactic acid units in such polylactides differs from the average sequence length of the D- and L-lactic acid units present in poly(meso-lactide) as described in detail below.

In case a polylactide a2) is present in the biodegradable polymer composition the minimum concentration is at least 1 wt.-%, based on the total weight of components a1) to e). The composition contains at maximum 48 wt.-%, preferably at maximum 40 wt.-%, more preferred at maximum 35 wt.-%, even more preferred at maximum 30 wt.-%, most preferred maximum 20 wt.-% and in particular preferred at maximum 10 wt.-% of at least one polylactide a2), based on the total weight of components a1) to e) of the biodegradable polymer composition. Polylactide a2) may also be absent, i.e. have the concentration 0 wt.-%.

Polylactide a2) has a number-average molecular weight as measured by GPC in THF against a polystyrene standard of at least 5000 g/mol, preferably at least 20 000 g/mol, more preferred at least 30 000 g/mol and most preferred more than 50 000 g/mol. Preferably, the upper limit of the number-average molecular weight is 200 000 g/mol. Preferably polylactide (a2) has a number-average molecular weight in the range of 5 000 g/mol to 200 000, more preferred the number-average molecular weights is in the range of 20 000 to 200 000 g/mol, even more preferred in the range of 30 000 to 130 000 g/mol, in particular in the range of 50 000 to 130 000 g/mol..

Polylactide a2) may have a relative viscosity of 1.1 to 6, such as 1.25 to 5, or 1.5 to 3.5, measured using a 1% wt/vol solution of the polylactic acid in chloroform against a chloroform standard on a capillary viscometer at 30°C.

Lactic units constitute at least 90%, preferably or at least 95% by weight of polylactide (a2). As with the PMLA, the remaining weight of polylactic acid a2) if any may include residues of an initiator compound and/or repeating units produced by polymerizing one or more monomers different from lactide.

The lactic units in polylactide a2) consist of L-lactic units and D-lactic units in a ratio of above 65:35 or below 35:65, more preferred in a ratio of at least 75:25 or at most 25:75. For ratios of > 65:35 this ratio may be 75:25 to 100:0, 80:20 to 100:0, 85:15 to 100:0, 86: 14 to 100:0. For ratios of < 35:65 this ratio may be 25:75 to 0:100, 20:80 to 0: 100, 15:85 to 0: 100, or 14:86 to 0:100. It is preferred that the L-lactic units and D-lactic units are arranged randomly.

The polylactide (a2) may be a semi- crystalline grade in which the ratio of L-lactic units to D-lactic units is 92:8 to 100:0 or 8:92 to 0: 100, or an amorphous grade in which the ratio of L-lactic units to D-lactic units is 86:14 to 92:8 or 8:92 to 14:86. The polylactide (a2) may be a homopolymer of L-lactide or a random copolymer of L-lactide with one or more of meso-lactide, D-lactide, and rac- lactide. In the latter case, the proportion of the various lactides is selected to provide a ratio of L-lactic units to D-lactic units above 65:35 to 99.9:0.1. This ratio may be 75:25 to 99.9:0.1, 80:20 to 99.9:0.1, 86: 14 to 99.9:0.1, 92:8 to 99.9:0.1 or 86:14 to 92:8.

Polylactide a2) is in some embodiments a homopolymer of D-lactide or a random copolymer of D-lactide with one or more of meso-lactide, L-lactide, and rac- lactide. In the latter case, the proportion of the various lactides is selected to provide a ratio of L-lactic units to D-lactic units below 35:65 to 0.1:99.9. This ratio in some embodiments is 25:75 to 0.1:99.9, 20:80 to 0.1:99.9, 14:86 to 0.1:99.9, 8:92 to 0.1:99.9 or 15:85 to 8:92.

Polylactide a2) preferably does not include both a polylactide in which the ratio of L-lactic units to D-lactic units above 65:35 and another polylactide in which the ratio of L-lactic units to D-lactic units is below 35:65.

The polylactide a2) preferably has an average block length of L-lactic units and/or of D-lactic units above 2.0.

The other characteristics of polylactide a2), and the manner in which it is manufactured, are as described above with regard to the PMLA and are described in WO 2020/251745A1.

The use of PLA as polylactide a2) having the following range of properties is particularly preferred:
- a melt volume rate (MVR) to EN ISO 1133 (190°C, 2.16 kg weight) of 0.5 to 30 especially 2 to 40 cm³/10 min;
- a melting point below 240° C;
- a water content of below 1000 ppm;
- a residual (lactide) monomer content of below 0.3%;
- a molecular weight M_{w} of above 80.000 daltons.

Examples of preferred polylactic acids are Ingeo^{®}, 6201D, 6202D, 6252D, 6060D, 3001D, 3052D, 3251D and especially Ingeo^{®} 4060D, 4032D, 4043D or 4044D polylactic acid (from NatureWorks).

Preferably the polylactide a2) is different from the poly(meso-lactide) a1).

The total concentration of poly(meso-lactide) a1) and polylactide a2) in the biodegradable polymer composition is 2 to 50 wt.-%, preferably the biodegradable polymer composition contains at least 4 wt.-%, more preferred at least 5 wt.-%, even more preferred at least 7 wt.-%, most preferred at least 9 wt.-%, and most preferred at least 13 wt.-% , and in particular at least 16 wt.-% of the polylactide composition a), based on the total weight of components a) to e) of the biodegradable polymer composition. The maximum concentration of poly(meso-lactide) a1) and polylactide a2) in the biodegradable polymer composition is 50 wt.-%, preferably 40 wt.-%, more preferred 30 wt.-%, even more preferred 25 wt.-%, most preferred 20 wt.-% and in particular preferred 18 wt.-%, based on the total weight of components a1) to e) of the biodegradable polymer composition. Preferred ranges of the total concentration of the poly(meso-lactide) a1) and the polylactide a2) in the biodegradable polymer composition are 4 to 40 wt.-%, more preferred 5 to 30 wt.-%, even more preferred 7 to 25 wt.-%, and most preferred 9 to 20 wt.-%, based on the total weight of components a1) to e) of the biodegradable polymer composition. The weight percentages are based on the total weight of components a1) to e). Depending on the intended use of the polymer composition different composition ranges may be more suited, see below.

Preferably the weight ratio of the poly(meso-lactide) a1) to the polylactide a2) is at least 1:3, more preferred at least 1:1, even more preferred at least 2:1.

### Biodegradable polyester b)

The biodegradable polymer composition contains as component b) at least 20 wt.-%, preferably at least 40 wt.-%, and more preferred at least 50 wt.-%, based on the total weight of components a1) to e) of the polymer composition, of at least one biodegradable polyester selected from aliphatic polyesters, aliphatic-aromatic polyesters and copolymers and mixtures thereof. The maximum concentration of component b) is 98 wt.-%, preferably 96 wt.-%, more preferred 95 wt.-% and even more preferred 93 wt.-%, based on the total weight of components a1) to e) of the polymer composition. Depending on the intended use of the polymer composition different composition ranges may be more suited, see below. Preferably the biodegradable polyester is selected from aliphatic-aromatic polyesters and mixtures of aliphatic-aromatic polyesters and aliphatic polyesters.

The biodegradable polymer composition may contain an aliphatic-aromatic polyester. This aliphatic-aromatic polyester is preferably derived from:
b-1) 20 to 70 mol %, based on the total amount of components b-1) and b-2), of at least one aliphatic C₄-C₁₈ dicarboxylic acid or C₄-C₁₈ dicarboxylic acid derivative;
b-2) 80 to 30 mol %, based on the total amount of components b-1) and b-2), of at least one aromatic dicarboxylic acid or aromatic dicarboxylic acid derivative;
b-3) 98 to 100 mol %, based on the total amount of b-1) and b-2), of a C₂-C₁₀ diol;
b-4) 0 to 2 wt%, based on the total weight of components b-1), b-2) and b-3), of an at least trifunctional branching agent; and
b-5) 0 to 2 wt%, based on the total weight of components b-1), b-2) and b-3), of a chain extender.

The aliphatic C₄ - C₁₈ dicarboxylic acids and C₄ - C₁₈ dicarboxylic acid derivatives b-1) are preferably selected from C₄ - C₁₄, more preferably from C₄ - C₁₃ dicarboxylic acids, their derivatives and mixtures thereof. The derivatives may be the C₁ - C₆ dialkyl esters or anhydrides. Examples of the C₁ - C₆ dialkylesters are dimethyl, diethyl, di-n-propyl, diisopropyl, di-n-butyl, diisobutyl, di-tert-butyl, di-n-pentyl, diisopentyl, and di n-hexylesters. Preferred are the C₁ -C₄ dialkyl esters and in particular preferred are dimethyl esters. An example of anhydrides is succinic acid anhydride. Preferably the aliphatic dicarboxylic acids and their derivatives are selected from succinic acid, 2-ethylsuccinic acid, glutaric acid, 2-methylglutaric acid, 3-methylglutaric acid, 2,2-dimethylglutaric acid, diglycolic acid, adipic acid, pimelic acid, suberic acid, azelaic acid, sebacic acid, dodecanedioic acid, brassylic acid, hexadecanedioic acid, octadecanedioic acid, oxaloace-tic acid, glutamic acid, aspartic acid, itaconic acid and maleic acid, their derivatives, in particular the C₁ -C₄ dialkyl esters, and mixtures thereof. More preferred the aliphatic C₄ - C₁₈ dicarboxylic acids are selected from succinic acid, adipic acid, azelaic acid, sebacic acid, 1,12-dodecanoic acid, brassylic acid, their derivatives, in particular the C₁ -C₄ alkyl esters, and mixtures thereof, in particular preferred the aliphatic dicarboxylic acids are selected from adipic acid, azelaic acid, sebacic acid and brassylic acid, their C₁ -C₄ alkyl esters, and mixtures thereof. Succinic acid, azelaic acid, sebacic acid, and brassylic acid have the additional advantage of being available from renewable raw materials.

The aliphatic C₄-C₁₈ dicarboxylic acids and their derivatives b-1) may be selected from mixtures of at least two aliphatic acids or their derivatives, e.g. from mixtures of adipic acid with one or more additional aliphatic acid selected from succinic acid, 2-methylsuccinic acid, glutaric acid, 2-methylglutaric acid, pimelic acid, suberic acid, azelaic acid, sebacic acid, brassylic acid, 1,12-dodecanedioc acid, hexadecanedioic acid, octadecane dioic acid, their anhydrides or their C₁ - alkyl esters. Preferred are mixtures of adipic acid and azelaic acid or their C₁ -C₄ alkyl esters, mixtures of adipic acid and sebacic acid or their C₁ -C₄ alkyl esters, mixtures of succinic acid and sebacic acid or their C₁ -C₄ alkyl esters, and mixtures of succinic acid and azelaic acid or their C₁ -C₄ alkyl esters. In case the mixture of the aliphatic acids or their derivatives comprises succinic acid or adipic acid, they are preferably present in amounts of 30 to 70 mol-%, based on the total amount of the aliphatic acids or their derivatives b-1).

The concentration of the one or more aliphatic C₄-C₁₈ dicarboxylic acid or C₄-C₁₈ dicarboxylic acid derivative b-1) is 20 to 70 mol %, based on the total weight of components b-1) and b-2).

The aromatic dicarboxylic acids or aromatic dicarboxylic acid derivatives b-2) are preferably selected from aromatic and heteroaromatic C₆-C₁₂ dicarboxylic acids, preferably from aromatic and heteroaromatic C₆-C₈ dicarboxylic acids, and their derivatives. Examples of such aromatic and heteroaromatic dicarboxylic acids and derivatives are terephthalic acid, isophthalic acid, 2,6-naphthoic acid and 1,5-naphthoic acid, 2,5-furandicarboxylic acid, 2,4-furandicarboxylic acid, 2,3-furandicarboxylic acid, 3,4-furandicarboxylic acid, their C₁-C₆ dialkyl esters, their anhydrides and mixtures thereof. Preferred are the C₁-C₄ dialkyl esters, in particular preferred are methyl esters. Examples of the C₁-C₆ -dialkylesters are dimethyl, diethyl, di-n-propyl, diisopropyl, di-n-butyl, diisobutyl, di-tert-butyl, di-n-pentyl, diisopentyl, and di-n-hexylesters. Preferably the aromatic dicarboxylic acids or their derivatives are selected from terephthalic acid, 2,5-furandicarboxylic acid and their derivatives, in particular their C₁-C₄ alkyl esters, in particular preferred are terephthalic acid and its C₁-C₄ alkyl esters.

The concentration of the aromatic dicarboxylic acid or aromatic dicarboxylic acid derivative is 80 to 30 mol %, based on the total amount of components b-1) and b-2). In case the aromatic dicarboxylic acid or dicarboxylic acid derivative is terephthalic acid or a derivative thereof, its concentration in the polyester is preferably 30 to 70 mol.-%, in case the aromatic dicarboxylic acid or dicarboxylic acid derivative is a furane dicarboxylic acid like 2,5-furandicarboxylic acid or a derivative thereof, its concentration in the polyester is preferably 50 to 80 mol.-%, based on the total amount of components b-1) and b-2).

The aliphatic diol b-3) is selected from aliphatic C₂-C₁₀ diols, preferably from C₂-C₆ diols and more preferred from C₂-C₄ diols. Examples of suitable aliphatic C₂-C₁₀ diols are 1,2-ethanediol, 1,2-propanediol, 1,3-propanediol, 1,2-butanediol, 1,4-butanediol, 1,5-pentanediol, 2,2-dimethyl-1,3-propanediol (neopentyl glycol), 1,6-hexanediol, 2,4-dimethyl-2-ethyl-1,3-hexanediol, 2,2-dimethyl-1,3-propanediol, 2-ethyl-2-butyl-1,3-propanediol, 2-ethyl-2-isobutyl-1,3 propanediol and 2,2,4-trimethyl-1,6-hexanediol, cyclopentanediol, 1,4-cyclohexanediol, 1,2-cyclohexanedimethanol, 1,3 cyclohexanedimethanol, 1,4-cyclohexanedimethanol, isosorbide, isoiodide and 2,2,4,4-tetramethyl-1,3 cyclobutanediol. Preferred aliphatic C₂-C₁₀ diols are 1,2-ethanediol, 1,3-propanediol, ,1,4-butanediol and 1,6-hexanediol. It is also possible to use mixtures of different aliphatic diols. Preferably the aliphatic diol b-3) contains at least 50% by moles of one or more diols selected from 1,2-ethanediol, 1,3-propanediol, 1,6-hexanediol and 1,4-butanediol. More preferred the aliphatic diol b-3) is 1,4-butanediol.

In particular preferred are diols generated from renewable resources like 1,4-butanediol from either direct fermentation (WO2008/115840) or from the hydrogenation of biobased succinic acid or 1,3-propanediol from fermentation developed by DuPont and Tate & Lyle.

The aliphatic diol b-3) is present in a concentration of 98 to 100 mol %, based on the based on the total amount of b-1) and b-2).

In particular preferred are aliphatic-aromatic polyesters wherein the aliphatic C₄-C₁₈ dicarboxylic acid and its derivative b-1) are selected from succinic acid, adipic acid, azelaic acid, sebacic acid, 1,12 dodecanedioic acid, brassylic acid, their derivatives, and mixtures thereof; wherein the aromatic dicarboxylic acid and aromatic dicarboxylic acid derivative b-2) are selected from terephthalic acid, 2,5-furandicarboxylic acid, their derivatives and mixtures thereof; and wherein the diol b-3) is 1,4-butanediol. The dicarboxylic acid derivatives are preferably the C₁ -C₄ alkyl esters, in particular the methyl esters.

The biodegradable polyester b) may contain a branching agent as component b-4), which contains at least three functional groups which are capable of reacting with a diol or a dicarboxylic acid. Examples are at least trihydric alcohols like glycerol, trimethylolpropane, trimethylolethane, pentaerythritol, polyethertriols and sorbitol or carboxylic acids and hydroxy acids or anhydrides containing three or more groups selected from carboxylic acid groups, carboxylic acid anhydride groups and hydroxy groups like tartaric acid, citric acid, malic acid, trimesic acid, trimellitic acid, trimellitic anhydride, pyromellitic and pyromellitic dianhydride, preferred are trimethylolpropane, pentaerythritol, and glycerol, in particular preferred are trimethylolpropane and glycerol. Component b-4) can be used to construct biodegradable polyesters having structural viscosity. Melt rheology improves in that the biodegradable polyesters become easier to process, for example easier to pull into self-supporting films/sheets by melt solidification.

The concentration of the at least trifunctional branching agent b-4) in the biodegradable polyester is 0 to 2 wt%, based on the total weight of components b-1), b-2) and b-3) in the final polyester. In case the at least trifunctional branching agent b-4) is present in the biodegradable polyester, the concentration is 0.01 to 2 wt%, preferably 0.05 to 1 wt%, and particular preferred 0.08 to 0.20 wt%, based on the total weight of components b-1), b-2) and b-3) in the final polyester.

The biodegradable polymer composition may contain as component b-5) a chain extender. Chain extenders are polyfunctional and especially difunctional isocyanates, isocyanurates, oxazolines, carboxylic anhydrides, carbodiimides or epoxides.

The term "epoxides" is to be understood as meaning particularly epoxy-containing copolymer based on styrene, acrylic ester and/or methacrylic ester, preferably of the styrene-glycidylether-methylmethacrylate type. The units which bear epoxy groups are preferably glycidyl (meth)acrylates. Copolymers having a glycidyl methacrylate content of greater than 20, more preferably greater than 30 and even more preferably greater than 50 wt% of the copolymer will be found particularly advantageous. The epoxy equivalent weight (EEW) in these polymers is preferably in the range from 150 to 3000 and more preferably in the range from 200 to 500 g/equivalent. The weight-average molecular weight MW of the polymers is preferably in the range from 2000 to 25 000 g/mol and particularly in the range from 3000 to 8000 g/mol. The number average molecular weight Mn of the polymers is preferably in the range from 400 to 6000 g/mol and particularly in the range from 1000 to 4000 g/mol. The polydispersity (Q) is generally between 1.5 and 5. Epoxy-containing copolymers of the abovementioned type are commercially available, for example from BASF Resins B.V. under the Joncryl^{®} ADR brand. Joncryl^{®} ADR 4468 and ADR 4400 areparticularly useful as chain extender.

Difunctional isocyanates may be aromatic or aliphatic diisocyanates.

Examples of aromatic diisocyanates are tolylene 2,4-diisocyanate, tolylene 2,6-diisocyanate, 2,2'-diphenylmethane diisocyanate, 2,4'-diphenylmethane diisocyanate, 4,4'-diphenylmethane diisocyanate, naphthylene 1,5-diisocyanate or xylylene diisocyanate. Of these, particular preference is given to 2,2'-, 2,4'- and also 4,4'-diphenylmethane diisocyanates. In general, the latter diisocyanates are used as a mixture. The diisocyanates may also comprise minor amounts, for example up to 5% by weight, based on the total weight, of urethione groups, for example for capping the isocyanate groups.

The term "aliphatic diisocyanate" herein refers particularly to linear or branched alkylene diisocyanates or cycloalkylene diisocyanates having 2 to 20 carbon atoms, preferably 3 to 12 carbon atoms, for example 1,6-hexamethylene diisocyanate, 1,5-pentamethylene diisocyanate, isophorone diisocyanate or methylenebis(4-isocyanatocyclohexane). Particularly preferred aliphatic diisocyanates are isophorone diisocyanate and, in particular, 1,6-hexamethylene diisocyanate.

The preferred isocyanurates include the aliphatic isocyanurates which derive from alkylene diisocyanates or cycloalkylene diisocyanates having 2 to 20 carbon atoms, preferably 3 to 12 carbon atoms, for example isophorone diisocyanate or methylenebis(4-isocyanatocyclohexane). The alkylene diisocyanates here may be either linear or branched. Particular preference is given to isocyanurates based on n hexamethylene diisocyanate, for example cyclic trimers, pen-tamers or higher oligomers of 1,6-hexamethylene diisocyanate.

2,2'-Bisoxazolines are generally obtainable via the process from Angew. Chem. Int. Ed., Vol. 11 (1972), pp. 287-288. Particularly preferred bisoxazolines are those in which R1 is a single bond, a (CH2)z alkylene group, where z = 2, 3 or 4, such as methylene, 1,2-ethanediyl, 1,3-propanediyl, 1,2-propanediyl or a phenylene group. Particularly preferred bisoxazolines are 2,2'-bis(2-oxazoline), bis(2-oxazolinyl)methane, 1,2-bis(2-oxazolinyl)ethane, 1,3-bis(2-oxazoli-nyl)propane or 1,4-bis(2-oxazolinyl)butane, in particular 1,4-bis(2-oxazolinyl)benzene, 1,2-bis(2-oxazolinyl)benzene or 1,3-bis(2-oxazolinyl)benzene.

Carbodiimides and polymeric carbodiimides are marketed by way of example by Lanxess with trademark Stabaxol^{®} or by Elastogran with trademark Elastostab^{®} or Carbodilite HMV-15CA and Carbodilite HMV-5CA-LC from Nisshinbo Chemical Inc.

Examples are N,N'-di-2,6-diisopropylphenylcarbodiimide, N,N'-di-o-tolylcarbodiimide, N,N'-di-phenylcarbodiimide, N,N'-dioctyldecylcarbodiimide, N,N'-di-2,6-dimethylphenylcarbodiimide, N-tolyl-N'-cyclohexylcarbodiimide, N,N'-di-2,6-di-tert-butylphenylcarbodiimide, N-tolyl-N'-phenyl-carbodiimide, N,N'-di-p-nitrophenylcarbodiimide, N,N'-di-p-aminophenylcarbodiimide, N,N'-di-p-hydroxyphenylcarbodiimide, N,N'-dicyclohexylcarbodiimide, N,N'-di-p-tolylcarbodiimide, p-phe-nylenebisdi-o-tolylcarbodiimide, p-phenylenebisdicyclohexylcarbodiimide, hexamethylenebis-dicyclohexylcarbodiimide, 4,4'-dicyclohexylmethanecarbodiimide, ethylenebisdiphenylcar-bodiimide, N,N'-benzylcarbodiimide, N-octadecyl-N'-phenylcarbodiimide, N-benzyl-N'-phenylcar-bodiimide, N octadecyl-N'-tolylcarbodiimide, N-cyclohexyl-N'-tolylcarbodiimide, N-phenyl-N'-tolyl-carbodiimide, N-benzyl-N'-tolylcarbodiimide, N,N'-di-o-ethylphenylcarbodiimide, N,N'-di-p-ethylphenylcarbodiimide, N,N'-di-o-isopropylphenylcarbodiimide, N,N'-di-p-isopropylphenylcar-bodiimide, N,N'-di-o-isobutylphenylcarbodiimide, N,N'-di-p-isobutylphenylcarbodiimide, N,N'-di-2,6-diethylphenylcarbodiimide, N,N'-di-2-ethyl-6-isopropylphenylcarbodiimide, N,N'-di-2-isobutyl-6-isopropylphenylcarbodiimide, N,N'-di-2,4,6-trimethylphenylcarbodiimide, N,N'-di-2,4,6-triisopropylphenylcarbodiimide, N,N'-di-2,4,6-triisobutylphenylcarbodiimide, diisopropylcarbodiimide, dimethylcarbodiimide, diisobutylcarbodiimide, dioctylcarbodiimide, tert-butylisopropyl-carbodiimide, di-β-naphthylcarbodiimide, di-tert-butylcarbodiimide, 1,6- hexamethylene bis(ethylcarbodiimide), 1,8-octamethylene bis(ethylcarbodiimide), 1,10-decamethylene bis(ethylcarbodiimide), 1,12 dodecamethylene bis(ethylcarbodiimide), p-phenylene bis(ethylcarbodiimide), poly(cyclooctylene carbodiimide), poly(I,4-dimethylencyclohexylene carbodiimide), poly(cyclohexylene carbodiimide), poly(ethylene carbodiimide), poly(butylene carbodiimide), poly(isobutylene carbodiimide), poly(nonylene carbodiimide), poly(dodecylene carbodiimide), poly(neopentylene carbodiimide), poly(I,4-dimethylene phenylene carbodiimide), poly(2,2',6,6'-tetraisopropyldiphenylene carbodiimide) (Stabaxol^{®} D), poly(2,4,6-triisopropyl-I,3-phenylene carbodiimide) (Stabaxol^{®} P- 100), poly(2,6 diisopropyl- 1,3-phenylene carbodiimide) (Stabaxol^{®} P), poly(tolyl carbodiimide), poly(4,4'- diphenylmethane carbodiimide), poly(3,3'-dimethyl-4,4'-biphenylene carbodiimide), poly(p- phenylene carbodiimide), poly(m-phenylene carbodiimide), poly(3,3'-dimethyl-4,4'- diphenylmethane carbodiimide), poly(naphthylene carbodiimide), poly(isophorone carbodiimide), poly(cumene carbodiimide), and p-phenylene bis(ethylcarbodiimide).

Preferably the chain extender b-5) is selected from isophorone diisocyanate, 1,6-hexamethylene diisocyanate, 1,5-pentamethylendiisocyanate, 4,4-diphenylmethane diisocyanate, and epoxy-containing copolymers based on styrene, acrylic ester and methacrylic ester, preferably of the styrene-glycidylether-methylmethacrylate type.

The concentration of the chain extender b-5) is 0 to 2 wt%, based on the total weight of components b-1), b-2) and b-3).

The number average molecular weight (Mn) of the aromatic-aliphatic polyesters used as component b) measured in a Hexafluoroisopropanol (HFIP) solution against narrow polymethylmethacrylate (PMMA) standards is generally in the range from 5 000 to 100 000, preferably in the range from 10 000 to 75 000 g/mol, and more preferred in the range from 15 000 to 50 000 g/mol, their weight average molecular weight (Mw) is generally in the range from 30 000 to 300 000, preferably 60 000 to 200 000 g/mol, and their Mw/Mn ratio is generally in the range from 1 to 6, preferably in the range from 2 to 4. The viscosity number is between 30 and 450 g/mL and preferably in the range from 50 to 400 g/mL Here and throughout the specification, the viscosity number (VN) is determined according to DIN 53728-3:1985-1 at 25 °C using a solution of the respective polymer in a 50:50 w/w mixture of phenol and 1,2- dichlorobenzene.. The melting point measured at 50% relative humidity at 23 °C by DSC with a heating rate of 20 °C is in the range from 85 to 150°C and preferably in the range from 95 to 140°C.

Polyesters suited for the use in the biodegradable polymer composition generally have a melt volume rate (MVR) to EN ISO 1133 (190°C, 2.16 kg weight) of 0.5 to 10.0 cm³/10 min and preferably of 0.8 to 5 cm³/10 min.

Examples of biodegradable aliphatic-aromatic polyesters are poly(butylene-co-succinate-co-terephthalate) ("PBST"), poly(butylene-co-adipate-co-terephthalate) ("PBAT"), poly(butylene-co-sebacate-co-terephthalate) ("PBSeT"), poly(butylene-co-azelate-co-terephthalate) ("PBAzT"), poly(butylene-co- sebacate-co-adipate-co-terephthalate) ("PBSeAT"), poly(butylene-co-azelate-co-adipinate-co-terephthalate), ("PBAzAT"), poly(butylene-co-azelate-co-succinate-co-terephthalate) ("PBAzST"), poly(butylene-co-azelate-co-2,5-furanoate) ("PBAzF"), and poly(butylene-co-sebacate-co-2,5-furanoate) ("PBSeF").

The biodegradable polyester b) may comprise at least one aliphatic polyester derived from:
b-1) at least one aliphatic C₄-C₁₈ dicarboxylic acid or C₄-C₁₈ dicarboxylic acid derivative;
b-3) 98 to 100 mol %, based on the total amount of b-1), of at least one aliphatic C₂-C₁₀ diol;
b-4) 0 to 2 wt%, based on the total weight of components b-1) and b-3), of an at least trifunctional branching agent; and
b-5) 0 to 2 wt%, based on the total weight of components b-1) and b-3), of a chain extender; or derived from:
   b-6) at least one C₂-C₁₈ hydroxycarboxylic acid or C₂-C₁₈ hydroxycarboxylic acid derivative; and
   b-4) 0 to 2 wt%, based on the total weight of component b-6), of an at least trihydric alcohol; and
   b-5) 0 to 2 wt%, based on the total weight of component b-6), of a chain extender;
   or a mixture thereof.

The components b-1), b-3), b-4) and b-5) of the aliphatic polyester are the same as described above and described as preferred for the aliphatic-aromatic polyester b).

Preferably the aliphatic polyesters are derived from a C₄-C₁₈ dicarboxylic acid b-1) selected from succinic acid, adipic acid, azelaic acid, sebacic acid, 1,12-dodecanedioic acid, and brassylic acid, their derivatives, and mixtures thereof; and the diol b-3) is 1,4-butanediol and the C₃-C₁₈ hydroxycarboxylic acid and its derivative b-6) is selected from glycolic acid, hydroxypropionic acid, hydroxybutanoic acid, hydroxyvaleric acid, hydroxyhexanoic acid, and caprolactone.

Within this specification, polylactide is not included in the aliphatic polyesters derived from b-6) and optionally b-4) and/or b-5).

Preferred aliphatic polyesters derived from components b-1), b-3), and optionally b-4) and/or b-5) are poly(butylene-co-succinate) ("PBS"), poly(butylene-co-sebacate) ("PBSe"), poly(butylene-co-azealate) ("PBAz") and poly(butylene-co-succinate-co-adipate) ("PBSA"), in case of PBSA the polyester preferably contains 13-23 mol-% adipic acid, based on the total amount of the acid component. In particular preferred are poly(butylene-co-succinate) and poly(butylene-co-succinate-co-adipate), wherein the PBSA preferably contains 13-23 mol-% adipic acid, based on the total amount of the acid component

Biodegradable aliphatic and aliphatic-aromatic polyesters as described above are commercially available, e.g. under the tradename ecoflex^{®} by BASF. An example is ecoflex^{®} F Blend C1200, which is a polybutylene(co-adipate-co terephthalate).

Component b-6) is selected from at least one C₂-C₁₈ aliphatic hydroxy carboxylic acid and C₃-C₁₈ hydroxycarboxylic acid derivative and mixtures thereof. Examples of such aliphatic hydroxy carboxylic acids or their derivatives are glycolic acid, hydroxypropionic acid, hydroxybutanoic acid, hydroxyvaleric acid, hydroxyhexanoic acid, hydroxydecanoic acid, hydroxydodecanoic acid, hydroxyhexadecanoic acid, hydroxyoctadecanoic acid, gamma-butyrolactone and caprolactone.

Preferred biodegradable polyesters derived from component b-6) and optionally components b-4) and/or b-5) are polycaprolactone and polyhydroxyalkanoates.

Polycaprolactone is usually prepared from caprolactone by ring opening polymerization. The molecular weight of the polycaprolactone is generally in the range from 40 000 to 100 000 g/mol and preferably in the range from 45 0000 to 85 000 g/mol determined via GPC in HFIP (hex-afluoro-2-propanol) as solvent against narrowly distributed PMMA standards.

The polyhydroxyalkanoates may be selected from polyhydroxybutyrates, polyhydroxybutyrate-valerates, polyhydroxybutyrate propanoates, polyhydroxybutyrate-hexanoates, polyhydroxybutyrate-decanoates, polyhydroxybutyrate-dodecanoates, polyhydroxybutyrate-hexadecano-ates, polyhydroxybutyrate-octadecanoates, and poly-3-hydroxybutyrate-4-hydroxybutyrates.

Poly-3-hydroxybutyrates are available from Tianan under the name Enmat^{®}. Poly(3-hydroxybutyrate-co-4-hydroxybutyrate)s were first developed by Metabolix. and will be commercialized by CJ CheilJedang. Poly(3-hydroxybutyrate-co-3-hydroxyhexanoate)s are commercially available from Kaneka (Aonilex^{™}) or Danimer Scientific (Nodax^{®}). Poly(3-hydroxybutyrate-co-3-hydroxyhexanoate)s generally have a 3-hydroxyhexanoate content of 1 to 20 and preferably 3 to 15 mol% based on the polyhydroxyalkanoate. Preferred are poly(hydroxybutyrate-co-hydroxyhexanoate)s, in particular poly(3-hydroxybutyrate-co-3-hydroxyhexanoate).

The molecular weight Mw of polyhydroxyalkanoates is generally in the range from 100 000 to 1 000 000 g/mol and preferably in the range from 300 000 to 600 000 g/mol, mol determined via GPC in HFIP (hexafluoro-2-propanol) as solvent against narrowly distributed PMMA standards.

Preferably the biodegradable polyester composition contains one or more aliphatic-aromatic polyester or a mixture of one or more aliphatic-aromatic polyester with one or more aliphatic polyester as component b). Particular preference is given to biodegradable polyester compositions containing an aliphatic-aromatic polyester selected from poly(butylene-co-succinate-co-terephthalate) ("PBST"), poly(butylene-co-adipate-co-terephthalate) ("PBAT"), poly(butylene-co-sebacate-co-terephthalate) ("PBSeT"), poly(butylene-co-azelate-co-terephthalate) ("PBAzT"), poly(butylene-co- sebacate-co-adipate-co-terephthalate) ("PBSeAT"), poly(butylene-co-azelate-co-adipinate-co-terephthalate) ("PBAzAT"), poly(butylene-co-azelate-co-succinate-co-terephthalate) ("PBAzST"), poly(butylene-co-azelate-co-2,5-furanoate) ("PBAzF"), poly(butylene-co-sebacate-co-2,5-furanoate) ("PBSeF"), mixtures of two or more of the aforementioned aliphatic-aromatic polyesters, and mixtures of one or more of the aforementioned aliphatic-aromatic polyesters with one or more aliphatic polyesters selected from polycaprolactone, poly(butylene-co-succinate), poly(butylene-co-succinate-co-adipate), and poly(3-hydroxybutyrate-co-3-hydroxyhexanoate)] as component b).

### Starch polymer c)

The biodegradable polymer composition may contain at least one starch polymer c). The term "starch polymer" as used herein means starch itself and polymers derived from starch.

Starch is a natural polymer composed of amylose and amylopectin. Amylose is essentially a linear polymer having a molecular weight in the range of 100,000-500,000, whereas amylopectin is a highly branched polymer having a molecular weight of up to several million. Although starch is produced in many plants, typical sources include seeds of cereal grains, such as corn, waxy corn, wheat, sorghum, rice, and waxy rice; tubers, such as potatoes; roots, such as tapioca (i.e., cassava and manioc), sweet potato, and arrowroot; and the pith of the sago palm. Broadly speaking, any natural (unmodified) and/or modified starch may be used as component c) in the biodegradable polymer composition. Modified starches, for instance, are often employed that have been chemically modified by typical processes known in the art (e.g., esterification, etherification, oxidation, acid hydrolysis, enzymatic hydrolysis, etc.). Starch ethers and/or esters may be particularly desirable, such as hydroxyalkyl starches, carboxymethyl starches, etc. The hydroxyalkyl group of hydroxylalkyl starches may contain, for instance, 2 to 10 carbon atoms, in some embodiments from 2 to 6 carbon atoms, and in some embodiments, from 2 to 4 carbon atoms. Representative hydroxyalkyl starches such as hydroxyethyl starch, hydroxypropyl starch, hydroxybutyl starch, and derivatives thereof. Starch esters, for instance, may be prepared using a wide variety of anhydrides (e.g., acetic, propionic, butyric, and so forth), organic acids, acid chlorides, or other esterification reagents. The degree of esterification may vary as desired, such as from 1 to 3 ester groups per glucosidic unit of the starch.

Thermoplastic starch contains a plasticizer to help render the starch melt-processible. Starches, for instance, normally exist in the form of granules that have a coating or outer membrane that encapsulates the more water-soluble amylose and amylopectin chains within the interior of the granule. When heated, plasticizers may soften and penetrate the outer membrane and cause the inner starch chains to absorb water and swell. This swelling will, at some point, cause the outer shell to rupture and result in an irreversible destructurization of the starch granule. Once destructurized, the starch polymer chains containing amylose and amylopectin polymers, which are initially compressed within the granules, will stretch out and form a generally disordered intermingling of polymer chains. Upon resolidification, however, the chains may reorient themselves to form crystalline or amorphous solids having varying strengths depending on the orientation of the starch polymer chains. Because the starch is thus capable of melting and resolidifying at certain temperatures, it is generally considered a "thermoplastic starch".

Suitable plasticizers may include, for instance, water, polyhydric alcohol plasticizers, such as sugars (e.g., glucose, sucrose, fructose, raffinose, maltodextrose, galactose, xylose, maltose, lactose, mannose, and erythrose), sugar alcohols (e.g., erythritol, xylitol, malitol, mannitol, and sorbitol), polyols (e.g., ethylene glycol, glycerol, poly glycerol, propylene glycol, dipropylene glycol, butylene glycol, and hexane triol), etc. In case the starch grain contains a sufficient high amount of water, it is also possible to use the water present in the starch grain as plasticizer. Also suitable are hydrogen bond forming organic compounds which do not have hydroxyl group, including urea and urea derivatives; anhydrides of sugar alcohols such as sorbitan; animal proteins such as gelatin; vegetable proteins such as sunflower protein, soybean proteins, cotton seed proteins; and mixtures thereof. Other suitable plasticizers may include phthalate esters, dimethyl and diethylsuccinate and related esters, glycerol triacetate, glycerol mono and diacetates, glycerol mono, di, and tripropionates, butanoates, stearates, lactic acid esters, citric acid esters, adipic acid esters, stearic acid esters, oleic acid esters, and other acid esters. Aliphatic acids may also be used, such as copolymers of ethylene and acrylic acid, polyethylene grafted with maleic acid, polybutadiene-co-acrylic acid, polybutadiene-co-maleic acid, polypropylene-co-acrylic acid, polypropylene-co-maleic acid, and other hydrocarbon based acids. A low molecular weight plasticizer is preferred, such as less than about 20,000 g/mol, preferably less than about 5,000 g/mol and more preferably less than about 1,000 g/mol. Preferred plasticizers are water, glycerol, oligo-glycerol, sorbitol and hydrogenated hydrolysed starch syrup (CAS 68425-17-2).

The relative amount of starches and plasticizers employed in the thermoplastic starch may vary depending on a variety of factors, such as the desired molecular weight, the type of starch, the affinity of the plasticizer for the starch, etc. Typically, however, starches constitute from about 30 wt. % to about 95 wt. %, in some embodiments from about 40 wt. % to about 90 wt. %, and in some embodiments, from about 50 wt. % to about 85 wt. % of the thermoplastic starch. Likewise, plasticizers typically constitute from about 5 wt. % to about 55 wt. %, in some embodiments from about 10 wt. % to about 45 wt. %, and in some embodiments, from about 15 wt. % to about 35 wt. % of the thermoplastic composition. Depending on the intended use of the polymer composition different composition ranges may be more suited, see below.

The starch polymer c) may be selected from flour, native starch, modified starch, hydrolyzed starch, destructured starch, gelatinized starch, plasticized starch, thermoplastic starch, biofiller comprising complexed starch, and mixtures thereof.

Preferably the starch polymer used as component c) is selected from native starches, more preferably from corn, potato, tapioca, pea, wheat or rice starch and most preferably from native corn or wheat starch, in particular preferred from corn.

Preferably the biodegradable polymer composition contains at least one starch polymer c). In this case the concentration of the starch polymer c) is usually at least 2 wt.-%, preferred at least 5 wt.-%, more preferred 10 wt.-%, even more preferred at least 25 wt.-%, most preferred at least 30 wt.-%, and in particular preferred at least 35 wt.-%, based on the total weight of the components a1) to e) of the biodegradable polymer composition. The maximum concentration of the starch polymer c) is usually 55 wt.-%, preferred 50 wt.-%, more preferred 45 wt.-%, based on the total weight of the components a1) to e) of the biodegradable polymer composition. Preferred concentration ranges of the starch polymer c) are 2 to 55 wt.-%, more preferred 10 to 50 wt.-%, even more preferred 25 to 50 wt.-%, most preferred 30 to 45 wt.-%, and in particular preferred 35 to 45 wt.-%, based on the total weight of the components a1) to e) of the biodegradable polymer composition. Depending on the specific field of application of the films prepared from the biodegradable polymer composition, different concentration ranges of the starch polymer c) may be preferred.

The weight of the starch polymer as used herein means the total weight of the starch polymer itself and an optionally present plasticizer but without water.

### Inorganic filler d)

The biodegradable polymer composition may contain at least one inorganic filler d) selected from salts of alkaline earth metals, silicic acids and their salts, silica gel, silicates, silicon dioxide (quartz), bentonite, graphite, carbon black, iron oxide, kaolin, sodium carbonate, titanium dioxide, wollastonite, mica, bentonite, montmorillonites, and mineral fibers.

Alkaline earth salts include sulfates like gypsum (CaSO₄ hydrate) in different forms like natural gypsum, natural anhydrite, gypsum prepared from exhaust gas; halogenides like calcium chloride; carbonates like dolomite (MgCa(CO₃)₂) or chalk (CaCO₃); phosphates like calcium phosphate, e.g. apatite, and monobasic, dibasic and tribasic phosphates of Mg; silicates; and hydrates of the aforementioned salts like talc (Mg₃Si₄O₁₀(OH)₂) and gypsum.

Preferred inorganic fillers d) are talc and CaCO₃, which can be used alone or in mixture.

In case an inorganic filler d) is present in the biodegradable polymer composition, the concentration of the inorganic filler is at least 2 wt.-%, preferably at least 5 wt.-% based on the total weight of the polymer composition. The maximum concentration of the inorganic filler is usually 40 wt.-%, preferably 35 wt.-% and more preferred 30 wt.-%, based on the total weight of the components a1) to e) of the polymer composition. Depending on the intended use of the polymer composition different composition ranges may be more suited, see below.

Calcium carbonate may be used for example at 10 to 30 wt%, preferably 10 to 28 wt% and more preferably 12 to 20 wt%, based on the total weight of the components a1) to e) of the polymer composition. Calcium carbonate from Omya will prove suitable inter alia. The average particle size of calcium carbonate measured with a Malvern Mastersizer X is generally in the range from 0.2 to 10 micrometers, preferably 0.5 to 5 and more preferably 0.5 to 2.5 micrometers.

Talc may be used for example at 3 to 15 wt%, preferably 5 to 10 wt% and more preferably 5 to 8 wt%, based on the total weight of the components a1) to e) of the polymer composition. Talc from Mondo Minerals will be found suitable inter alia. The average particle size of talc is generally 0.5-10, preferably 1-8 and more preferably 1-3 micrometers when measured with a Sedi-graph 51XX.

### Additives - component e)

The biodegradable polymer composition may contain one or more further additives selected from cross-linking agents, lubricants, chain extenders, stabilizers, nucleating agents, slip and release agents, surfactants, waxes, antistatic agents, antifoggants, anti-block agents, dyes, pigments, UV absorbers, UV stabilizers and other plastics additives as component e).

Examples of branching agents and chain extenders are the compounds listed under b-4) and b-5), preferred branching agents and chain extenders are epoxy-containing copolymer based on styrene, acrylic ester and methacrylic ester, preferably of the styrene-glycidylether-methylmethacrylate type, and carbobdiimides, in particular preferred are epoxy-containing copolymer based on styrene, acrylic ester and methacrylic ester, preferably of the styrene-glycidylether-methylmethacrylate type . Preferably the biodegradable polymer composition contains 0.05 to 1 wt.-% by weight, preferably 0.05 to 0.2 wt.-%, based on the total weight of the components a1) to e) of the biodegradable polymer composition, of an epoxy-containing copolymer based on styrene, acrylic ester and/or methacrylic ester. Epoxy-containing copolymers of the abovementioned type are commercially available, for example from BASF Resins B.V. under the Joncryl^{®} ADR brand. Joncryl^{®} ADR 4468 and Joncryl^{®} ADR 4400 are particularly suitable.

Examples of slip and release agents are C₁₈-C₂₄-carboxamide such as stearamide, oleamide, erucamide and behenamide, and stearates like calcium stearate. Preferably the biodegradable polymer composition contains 0.05 wt.-% to 1 wt.-%, based on the total weight of the components a1) to e) of the biodegradable polymer composition, of a C₁₈-C₂₄-carboxamide, preferably selected from stearamide, erucamide, and behenamide or mixtures thereof.

Examples of surfactants are polysorbates, palmitates and laurates.

Examples of UV absorbers are 2-(4,6-bis-biphenyl-4-yl-1,3,5-triazin-2-yl)-5-(2-ethyl-(n)-hex-yloxy)phenol and carbon black. Preparation and properties of said UV absorber are known from WO 2009/071475.

Component e) is generally employed in concentrations of 0 to 5 wt.-%, preferably in in concentrations of 0 to 2 wt.-%, more preferred 0.05 to 2 wt.-%, even more preferred 0.05 to 1 wt.-% and in particular preferred 0.1 to 1 wt.-%, based on the total weight of the components a1) to e) of the biodegradable polymer composition.

In case the biodegradable polymer composition does not contain a starch polymer c) the composition usually contains at least 2 wt.-%, preferably at least 5 wt.-%, more preferred at least 9 wt.-%, even more preferred at least 15 wt.-%, and most preferred at least 20 wt.-% and usually up to 50 wt.-%, preferably up to 45 wt.-%, more preferred up to 40 wt.-%, and in particular preferred up to 35 wt.-%components a1) and a2) and usually at least 50 wt.-%, preferably at least 55 wt.-%, more preferred at least 60 wt.-% and in particular preferred at least 65 wt.-% of at least one biodegradable aliphatic or aliphatic-aromatic polyester b) and up to 98 wt.-%, 95 wt.-%, 91 wt.-%, 85 wt.-% 80 wt.-% of at least one biodegradable aliphatic or aliphatic-aromatic polyester b), based on the total weight of components a1) to e). Such biodegradable polymer compositions may contain
a) 2 to 50 wt.-%, preferably 5 to 50 wt.-%, more preferred 9 to 50 wt.-%, even more preferred 15 to 45 wt.-% and in particular preferred 20 to 35 wt.-%, based on the total weight of components a1), to e) of the polymer composition, of poly(meso-lacide) a1) and polylactide a2), wherein the minimum concentration of the poly(meso-lacide) a1) is 2 wt.-%, preferably 4 wt.-%, more preferred 5 wt.-%, even more preferred 7 wt.-%, most preferred 9 wt.-% and in particular 13 wt.-%, based on the total weight of components a1) to e);
b) 50 to 98 wt.-%, preferably 50 to 95 wt.-%, more preferred 50 to 91 wt.-% 55 to 85 wt.-%, an in particular preferred 65 to 80 wt.-%, based on the total weight of components a1), a2) to e) of the polymer composition, of at least one biodegradable aliphatic or aliphatic-aromatic polyester;
c) 0 wt.-% of at least one starch polymer;
d) 0 to 30 wt.-%, based on the total weight of components a), b), d) and e) of the polymer composition, of at least one inorganic filler; and
e) 0 to 5 wt%, based on the total weight of components a), b), d) and e) of the polymer composition, of at least one compound selected from cross-linking agents, lubricants, chain extenders, stabilizers, nucleating agents, slip and release agents, surfactants, waxes, antistatic agents, antifoggants, dyes, pigments, UV absorbers, UV stabilizers and other plastics additives.

In case the biodegradable polymer composition contains at least one starch polymer c) the biodegradable polymer composition may contain
a1) 2 to 40 wt.-%, based on the total weight of components a1) to e) of the polymer composition, of poly(meso-lactide);
a2) 0 to 38 wt.-%, based on the total weight of components a1) to e) of the polymer composition, of polylactide a2), wherein the weight percentages of components a1) and a2) is at maximum 40 wt.-%;
b) 20 to 96 wt.-%, based on the total weight of components a1) to e) of the polymer composition, of at least one biodegradable aliphatic or aliphatic-aromatic polyester;
c) 2 to 55 wt.-%, based on the total weight of components a1) to e) of the polymer composition, of at least one starch polymer;
d) 0 to 30 wt.-%, based on the total weight of components a1) to e) of the polymer composition, of at least one inorganic filler; and
e) 0 to 5 wt%, based on the total weight of components a1) to e) of the polymer composition, of at least one compound selected from cross-linking agents, lubricants, chain extenders, stabilizers, nucleating agents, slip and release agents, surfactants, waxes, antistatic agents, antifoggants, dyes, pigments, UV absorbers, UV stabilizers and other plastics additives.

A preferred biodegradable polymer composition contains
a1) 2 to 30 wt.-%, preferably 5 to 25 wt.-%, more preferred 9 to 20 wt.-%, based on the total weight of components a1) to e) of the polymer composition, of at least one poly(meso-lactide);
a2) 0 to 28 wt.-%, based on the total weight of components a1) to e) of the polymer composition, of at least one polylactide a2), wherein the weight percentages of the poly(meso-lactide) a1) and the polylactide a2) is at maximum 30 wt.-%;
b) 40 to 96 wt.-%, based on the total weight of components a1) to e) of the polymer composition, of at least one biodegradable aliphatic or aliphatic-aromatic polyester;
c) 2 to 30 wt.-%, preferably 5 to 30 wt.-%, more preferred 10 to 25 wt.-%, based on the total weight of components a1) to e) of polymer composition, of at least one starch polymer;
d) 0 to 30 wt.-%, based on the total weight of components a1) to e) of the polymer composition, of at least one inorganic filler; and
e) 0 to 5 wt%, based on the total weight of components a1) to e) of the polymer composition, of at least one compound selected from cross-linking agents, lubricants, chain extenders, stabilizers, nucleating agents, slip and release agents, surfactants, waxes, antistatic agents, antifoggants, dyes, pigments, UV absorbers, UV stabilizers and other plastics additives.

This biodegradable polymer composition is particularly suited for the preparation of mulch films. Mulch films benefit from a higher value of the elongation at maximum tensile strength in machine direction, since this facilitates the application of the mulch film on the fields.

Another preferred biodegradable polymer composition contains
a1) 2 to 40 wt.-%, preferably 5 to 30 wt.-%, more preferred 9 to 25 wt.-%, based on the total weight of components a1) to e) of the polymer composition, of at least one polylactide composition;
a2) 0 to 38 wt.-% based on the total weight of components a1) to e) of the polymer composition, of at least one polylactide a2), wherein the weight percentages of the poly(meso-lactide) a1) and the polylactide a2) is at maximum 40 wt.-%;
b) 20 to 73 wt.-%, based on the total weight of components a1) to e) of the polymer composition, of at least one biodegradable aliphatic or aliphatic-aromatic polyester;
c) 25 to 55 wt.-%, preferably 25 to 50 wt.-%, more preferred 30 to 45 wt.-%, based on the total weight of components a1) to e) of the polymer composition, of at least one starch polymer;
d) 0 to 30 wt.-%, based on the total weight of components a1) to e) of the polymer composition, of at least one inorganic filler; and
e) 0 to 5 wt%, based on the total weight of components a1) to e) of the polymer composition, of at least one compound selected from cross-linking agents, lubricants, chain extenders, stabilizers, nucleating agents, slip and release agents, surfactants, waxes, antistatic agents, antifoggants, dyes, pigments, UV absorbers, UV stabilizers and other plastics additives.

This biodegradable polymer composition is particularly suited for the preparation of thin films, e.g. 6 to 40 micrometer, which are widely used for carrier bags like shopping bags and for bags for fruits and vegetables and for waste bags, in particular organic waste bags. Such bags having a sufficiently high tear resistance in transversal direction are preferred.

Another preferred biodegradable polymer composition contains
a1) 2 to 20 wt.-%, preferably 2 to 10 wt.-%, based on the total weight of components a1) to e) of the polymer composition, of at least one poly(meso-lactide);
a2) 0 to 18 wt.-%, based on the total weight of components a1) to e) of the polymer composition, of at least one polylactide a2), wherein the weight percentages of the poly(meso-lactide) a1) and the polylactide a2) is at maximum 20 wt.-%;
b) 55 to 98 wt.-%, based on the total weight of components a1) to e) of the polymer composition, of at least one biodegradable aliphatic or aliphatic-aromatic polyester;
c) 0 to 10 wt.-%, based on the total weight of components a1) to e) of the polymer composition, of at least one starch polymer;
d) 0 to 10 wt.-%, based on the total weight of components a1) to e) of the polymer composition, of at least one inorganic filler; and
e) 0 to 5 wt%, based on the total weight of components a1) to e) of the polymer composition, of at least one compound selected from cross-linking agents, lubricants, chain extenders, stabilizers, nucleating agents, slip and release agents, surfactants, waxes, antistatic agents, antifoggants, dyes, pigments, UV absorbers, UV stabilizers and other plastics additives.

This biodegradable polymer composition is particularly suited for as cling film which require outstanding transparency and high tear resistance values.

Another object of the present invention is the use of the biodegradable polymer composition as described herein for manufacture of films and films comprising the biodegradable polymer composition as described. Such films may be produced by the processes known by person skilled in the art, e.g. by mono- or multilayer blown-film extrusion or mono- or multilayer flat-film extrusion. Additionally, they might be stretched either mono- or biaxially in order to improve their mechanical properties and transparency even further.

The biodegradable polymer composition can be used for the production of packaging, e.g. carrier bags like shopping bags, fruit and vegetable bags, cling films, but also for waste bags, in particular organic waste bags. It can also be used for the production of films used in agriculture like mulch films. Another object of the present invention is packaging comprising a film comprising the biodegradable polymer composition as described herein, e.g. carrier bags like shopping bags and bags for fruits and vegetables and waste bags, in particular organic waste bags. A further object of the present invention are mulch films comprising a film comprising the biodegradable polymer composition as described herein.

The films comprising the biodegradable polymer composition may consist of one layer (also called monolayer film) or may comprise two, three or more layers (also called multilayer films). Such multilayer film may comprise one or more layers of the same biodegradable polymer composition or more than one layer of different biodegradable polymer compositions as described above. The multilayer films may also comprise additional layers prepared from polymers or polymer composition different from the biodegradable polymer composition described above. E.g. an additional layer may be made by an aliphatic or aliphatic-aromatic polyester as described above as component b). Examples of such two- and three layer films are films having the following layer sequences, wherein A and A' denote different biodegradable polymer compositions as described above and B denotes a polymer composition different from the biodegradable polymer compositions according to the invention: A/B, A/B/A, B/A/B, A/A', A/A'/A, A/B/A', and A/A'/B.

The films have preferably a thickness in the range from 5 to 50 micrometer, preferably in the range from 6 to 40 micrometer. Films to be used as bags, e.g. as thin carrier bags or bags for fruits and vegetables preferably have a film thickness in the range from 7 to 30 micrometer. For thin carrier bags the film thickness is preferably in the range from 15 to 30 micrometer, for bags for fruits and vegetables the film thickness is preferably in the range from 7 to 15 micrometer. In case the film is intended to be used as mulch film, the film thickness is preferably in the range from 6 to 20 micrometer.

For the purposes of the present invention, the feature "biodegradable" for a substance or a mixture of substances is fulfilled if this substance or the mixture of substances according to DIN EN 13432 has a percentage degree of biodegradation of at least 90% after 180 days.

In the following the invention is described by examples which are to be interpreted merely as a descriptive enclosure which in no way has any limiting effect at all.

### A) Examples without starch polymer:

### Raw materials:

A1: ecoflex^{®} F C1200 (BASF), a poly(butylene-co-adipate-co-terephthalate) with approximately 47 mol% of terephthalic acid and a Melt Volume Rate at 190 °C / 2.16 kg of 3.5 cm³/10min.
B1: Ingeo^{®} 4043D (NatureWorks), a polylactic acid having a D-Lactic acid content of approximately 4% and a relative viscosity of 4.0 (see Table 1 for details).
B2: Ingeo^{®} 4060D, a polylactic acid (NatureWorks) having a D-Lactic acid content between 11-13% and a relative viscosity of 3.5 (see Table 1 for details).
B3: Poly(meso-lactide) having a relative viscosity of 2.0 obtained from Natureworks LLC (see Table 1 for details)
B4: Poly(meso-lactide) having a relative viscosity of 2.7 obtained from Natureworks LLC (see Table 1 for details)
B5: Poly(meso-lactide) having a relative viscosity of 2.9 obtained from Natureworks LLC (see Table 1 for details)

All polylactides B1 to B5 contained ≥ 99.5 % polylactide.
C1: ecoflex^{®} Batch AB1 (Antiblock batch commercially available by BASF on basis of ecoflex^{®} C1200 containing 60% CaCO₃ )
C2: ecoflex^{®} Batch FBA (20 wt.-% Joncryl^{®} ADR 4468 predispersed in 80 wt.-% ecoflex^{®} C1200)
C3: ecoflex^{®} Batch SL 10B (10 wt.-% behenamide predispersed in 90 wt.-% ecoflex^{®} C1200; slip agent masterbatch commercially available by BASF)
D1: CaCO₃
D2: Talc

**Table 1 Detailed analytical data for polylactides B1 and B2 and the poly(meso lactides) B3 to B5**

| Raw material | relative viscosity | D-Lactic acid content / % | L-Lactic acid content / % | Mn / Mw from GPC / g/mol | Glass transition temperature / °C | |
|---|---|---|---|---|---|---|
| | | | | | At 0% relative humidity | At 100% relative humidity |
| B1 | 4.0 | 4 | 96 | 105 000 / 209 000 | 63 | 51 |
| B2 | 3.5 | 11-13 | 87-89 | 99 000/ 191 000 | 61 | 49 |
| B3 | 2.0 | 45.2 | 54.8 | 62 000 / 113 000 | 53 | 42 |
| B4 | 2.7 | 44.7 | 55.3 | 94 000 / 193 000 | 53 | 42 |
| B5 | 2.9 | 43.9 | 56.1 | 107 000 / 215 000 | 53 | 42 |

### Determination of relative viscosity:

The relative viscosity of the poly lactides or poly (meso lactides) are measured using a 1% wt/vol solution of the polylactide resin in chloroform (prepared at 50 °C for 2 hours) against a chloroform standard on a capillary viscometer Viscotec Y501 at 30°C.

Determination of molecular weight of poly(meso lactides) by Gel Permeation Chromatography: The molecular weight of the poly (meso lactides) was measured by GPC using THF as solvent against narrow polystyrene standards. 0.50 g of PLA sample were dissolved in 9.2 mL of dichloromethane and shaken until dissolved. 0.25 mL of the solution were pipetted into a 20 mL vial and 4.75 mL of THF were added in order to adjust the concentration to 0.25 % wt./vol. 100 microliter of the solution were injected into the GPC using THF in isocratic mode at 1.0 mL / min flow rate. Detection was performed using a RI detector.

### Determination of glass transition temperature:

The glass transition temperature of the poly (meso lactides) was measured after conditioning the polymer at 0 and 100 % relative humidity by differential scanning calorimetry at a temperature ramping rate of 20°C/minute. For conditioning about 5 mg of the sample in a 40 µL aluminium sample pan and lid were melted at 180°C, followed by a quench to erase heat history and to ensure contact with the pan. The measurement was done with a Mettler DSC 3+. The midpoint of the transition slope was taken as the glass transition temperature.

### Determination of relative amounts of D- and L-Lactic acid enantiomers:

2.00 g of the polylactide or poly(meso-lactide) samples were stirred at 65 °C for 30 min in 40 mL of a 1 molar methanolic KOH solution. 3 ml concentrated sulfuric acid were carefully added in order to lower the pH into the acidic pH region and esterification with methanol was conducted for 10 min at 65 °C. To 1.25 mL of the obtained solution 0.75 mL of water and 3.25 mL of dichloromethane were added and the organic phase are separated after mixing. The dichloromethane phase was analyzed by chiral GC using an Agilent CycloSil-B chiral capillary GC column which contained 30% loading of b-cyclodextrin derivative embedded in DB-1701 mid polarity stationary phase. Methyl lactate enantiomers elute under isothermal conditions and the ratio was directly calculated from the peak areas since both enantiomers exhibit the same response factors.

Compositions were prepared according to the concentrations shown in Table 2. The concentrations are given in wt.-%, based on the total weight of the composition. IE means inventive example, CE means comparative example.

**Table 2 Compositions of IE 1 to IE 3 and CE 1 to CE 6**

| | IE 1 | CE 1 | CE 2 | IE 2 | CE 3 | CE 4 | IE 3 | CE 5 | CE 6 |
|---|---|---|---|---|---|---|---|---|---|
| A1 | 84.5 | 84.5 | 84.5 | 75 | 75 | 75 | 67.5 | 67.5 | 67.5 |
| B1 | | 9 | | | 18 | | | 8 | |
| B2 | | | 9 | | | 18 | | | 8 |
| B4 | 9 | | | 18 | | | 8 | | |
| C1 | 4 | 4 | 4 | 4 | 4 | 4 | | | |
| C2 | 0.5 | 0.5 | 0.5 | 1 | 1 | 1 | 0.5 | 0.5 | 0.5 |
| C3 | 2 | 2 | 2 | 2 | 2 | 2 | 2 | 2 | 2 |
| D1 | | | | | | | 14 | 14 | 14 |
| D2 | | | | | | | 6 | 6 | 6 |

### Manufacture of the blends without starch:

The blends were produced on a co-rotating twin screw extruder Coperion ZSK 26 MC having 11 barrels and a length L = 44D. All raw materials were dosed using individual gravimetric feeders. Granules A, B and C were fed to the cold zone 1 of the extruder and subsequently melted. The powdered mineral fillers were fed into the melt using a side feeder, subsequently mixed with the melt and homogenized. The melt was formed into strands at the die head, cooled in a water bath and granulated. The granules were dried until they reached a water content of less than 500 ppm.
Temperature profile [°C]: 30 - 180 - 9 x 210
Screw speed: 300 rpm
Throughput: 20 kg/h

### Description of film blowing:

The granules were fed into a 30 mm single screw extruder having a length of 25D and extruded through a spiral mandrel distributer from company Reifenhäuser having a die gap of 0.8 mm, a die diameter of 80 mm and a cooling ring. The blow-up ratio of the film was adjusted to 3.5 which corresponds to a lay flat width of 44 cm.
Temperature profile [°C]: water cooled-150-160-170 - afterwards all zones 180 (adapter until die)
Screw speed of the extruder: 80 U/min

### Description of analytics

### Determination of residual moisture:

The residual moisture was determined by Karl-Fischer-Titration using a Mettler-Toledo InMotion KF PRO Oven Autosampler at 130 °C heating temperature.

### Melt-Volume-Rate (MVR):

The melt volume rate (MVR) was measured according to EN ISO 1133 at the specified temperatures and weights and are listed in cm³ / 10 min.

### Mechanical Testing

### Film thickness:

The film thickness was measured with a digital thickness gauge from Mitutoyo (resolution 0,001 mm) at several spots of the film and the average value rounded to the next micron is reported.

### Tear resistance (Pendulum Elmendorf-Test)

The tear resistance of the films was determined according to EN ISO 6383-2:2004 using a Pro-Tear^{®} Electronic Elmendorf Tear Tester from Thwing-Albert Instrument Company and constant-radius film samples (43 mm tear length) under norm conditions (23 °C, 50% relative humidity).

### Mechanical data from tensile testing:

The E-Modulus, maximum tensile strength and elongation at maximum tensile strength were obtained from the blown films according to ISO 527-3:2018 under norm conditions (23 °C, 50% relative humidity). For each value 5 specimen type 2 with a width of 15 mm and a length of 150mm were measured. The initial distance between the grips was 50mm. The value for the E-Modulus was obtained at a test speed of 1mm/min. The other parameters were obtained at a test speed of 125 mm/min.

### Optical Parameters:

The values for the total light transmittance, Haze (all measured according to ASTM D1003 -13) and clarity were measured using a Haze-Gard Plus instrument from BYK-Gardner GmbH. The lower the Haze values the better whereas for clarity it is the opposite.

The results of the mechanical testing and the optical parameters are shown in Table 3.

**Table 3 Results of the mechanical testing of IE 1 to IE3 and CE1 to CE6**

| | **IE** 1 | **CE 1** | **CE 2** | **IE 2** | **CE 3** | **CE 4** | **IE 3** | **CE 5** | **CE6** |
|---|---|---|---|---|---|---|---|---|---|
| **Film thickness / micron µm** | 12 | 12 | 12 | 12 | 12 | 12 | 11 | 11 | 11 |

| **Tensile test machine direction** | | | | | | | | | |
|---|---|---|---|---|---|---|---|---|---|
| **E-Modulus / MPa** | 275 | 291 | 347 | 519 | 561 | 562 | 318 | 390 | 373 |
| **Max. tensile strength / MPa** | 34.4 | 36.5 | 38.4 | 36.5 | 32.6 | 33.1 | 31.2 | 32.4 | 32.0 |
| **Elongation at maximum tensile strength / %** | 253 | 210 | 257 | 277 | 173 | 182 | 225 | 155 | 165 |

| **Tensile test transversal direction** | | | | | | | | | |
|---|---|---|---|---|---|---|---|---|---|
| **E-Modulus / MPa** | 176 | 167 | 188 | 281 | 261 | 270 | 218 | 239 | 232 |
| **Max. tensile strength / MPa** | 24.8 | 30.2 | 27.8 | 21.3 | 31.4 | 25.9 | 17.3 | 21.1 | 19.1 |
| **Elongation at maximum tensile strength / %** | 488 | 499 | 517 | 459 | 427 | 427 | 411 | 417 | 439 |

| **Tear resistance** / **mN** | | | | | | | | | |
|---|---|---|---|---|---|---|---|---|---|
| **machine direction** | 99 | 898 | 1129 | 85 | 79 | 576 | 124 | 1294 | 1138 |
| **transversal direction** | 1149 | 380 | 467 | 1180 | 199 | 337 | 1269 | 412 | 505 |

| **Transparency** | | | | | | | | | |
|---|---|---|---|---|---|---|---|---|---|
| **Total transmittance / %** | 92.6 | 91.7 | 92.2 | | | | | | |
| **Haze / %** | 27.4 | 43.4 | 33.0 | | | | | | |
| **Clarity / %** | 70.3 | 54.6 | 64.4 | | | | | | |

Examples IE 1, IE 2 and CE 1 to CE 4 show that the use of poly(meso-lactide) in a composition containing no fillers leads to distinct higher values of the tear resistance in transversal direction and also to a reversion of the values of the tear resistance in machine direction and transversal direction. The films containing poly(meso-lactide) have a higher tear resistance in transversal direction at a concentration of 9 wt.-% and even 18 wt.-%. In contrast, the commonly used PLA composed of at least 87 % L-lactic units and 11-13 % D-lactic units shows already at a concentration of 9 wt.-% lower values for the tear resistance in transversal direction as well as at 18 wt.-%. Additionally, the elongation at maximum tensile strength in machine direction does not decrease by doubling the content of the poly(meso-lactide), whereas the films containing commonly used PLA instead of the poly(meso-lactide) show a significant decrease. The same effects occur with the films of IE 3 and CE 5 and CE 6, which additionally contain a mineral filler. It can also be noticed that adding an inorganic filler increases the values of the tear resistance both in machine direction and transversal direction.

Furthermore, the film containing the poly (meso lactide) has a significantly improved transparency compared to films using the common PLA. This is proven by the lower Haze value of IE 1 when compared to CE 1 and CE 2 and also the higher clarity of IE 1 compared to CE 1 and CE 2.

### B) Examples containing starch

### Raw materials

The compounds A1, A2, B1 to B5 and C1 are the same as described above. Additional compounds used for the starch containing compositions were
C4: ecoflex^{®} Batch SL 10C (10 wt.-% stearamide predispersed in 90 wt.-% ecoflex^{®} C1200; slip agent masterbatch commercially available by BASF)
D: Native corn starch, water content 12 wt.-%
E: Neosorb 70/70, 70 wt.-% hydrogenated hydrolyzed starch sirup dissolved in water supplied by Roquette

Compositions were prepared according to the concentrations shown in Table 4. The concentrations are given in wt.-%, based on the amounts of compounds A1 to C2 plus the TPS in the dry composition, i.e. the combined weight of the starch D and the plasticizer E without the water initially present in starch D and plasticizer E before feeding into the extruder. IE means inventive example, CE means comparative example. TPS means thermoplastic starch which was produced during the extrusion process from D and E.

**Table 4 Compositions of IE 4 to IE 7 and CE 7 to CE 13**

| | **CE 7** | **CE 8** | **CE 9** | **IE 4** | **IE 5** | **CE 10** | **CE 11** | **CE 12** | **CE 13** | **IE 6** | **IE 7** |
|---|---|---|---|---|---|---|---|---|---|---|---|
| **A1** | 58 | 50.5 | 50.5 | 50.5 | 50.5 | 59.5 | 49 | 44.5 | 44.5 | 44.5 | 49 |
| **B1** | | 7 | | | | 10 | 20 | 13 | | | |
| **B2** | | | 7 | | | | | | 13 | | |
| **B3** | | | | 7 | | | | | | | |
| **B4** | | | | | 7 | | | | | | |
| **B5** | | | | | | | | | | 13 | 18 |
| **C1** | | 0.5 | 0.5 | 0.5 | 0.5 | 0.5 | 1 | 0.5 | 0.5 | 0.5 | 1 |
| **C4** | 2 | 2 | 2 | 2 | 2 | 2 | 2 | 2 | 2 | 2 | 2 |
| **D** | 34 | 34 | 34 | 34 | 34 | 23.7 | 23.7 | 36.4 | 36.4 | 36.4 | 25.5 |
| **E** | 14.4 | 14.4 | 14.4 | 14.4 | 14.4 | 10.2 | 10.2 | 11.3 | 11.3 | 11.3 | 10.9 |
| | | | | | | | | | | | |
| **TPS in the dry com-positon (starch + plasticizer)** | 40 | 40 | 40 | 40 | 40 | 28 | 28 | 40 | 40 | 40 | 30 |

### Manufacture of the blends with starch:

The blends containing starch were produced according to the procedure described in WO2020156970A1 using a co-rotating twin screw extruder Coperion ZSK 45 MC¹⁸ with a diameter of 45 mm and 15 x 4D barrels resulting in a total length of L = 60D. The specific torque of the extruder was 18 Nm/cm³.

All components were dosed using separate gravimetric feeders or pumps. The starch D was dosed into barrel 1 and the liquid plasticizer E into barrel 2. All other components were dosed into barrel 5 by means of a side feeder ZS-B from Coperion and were subsequently melted, plasticized and homogenized. After the water and other volatiles were removed the granulation of the melt was performed using an underwater pelletizing equipment. The water content was below 0.5 % in all cases and no subsequent drying was necessary.
Temperature profile [°C]: 30-30-30-30-30-90-120-160-160-160-160-160-160-160-160- 2 x 160 (2 flanges) - 180 (start-up valve) - 180 (die plate).
Screw speed: 350 rpm
Throughput: 175 kg/h (calculated on basis of the dry end product)

The films were formed using the same film blowing machine described previously. Temperature profile [°C]: water cooled-120-170-170-afterwards all 170 (from flange until die)

### Mechanical Testing

### Film thickness:

The film thickness for starch containing films was calculated from their known density (1.29 g/cm³ at 30% thermoplastic starch content, 1.31 g/cm³ at 40% thermoplastic starch content) and the weight of an exactly 100 cm² big film piece in analogy to the method described for polyethylene in Annex 3 of ASTM E252- 06. The thickness was rounded to the next micron.

The tear resistance of the blown films and the mechanical values from the tensile testing were determined as described previously for the blends without starch.

The mechanical testing was carried out as described above. The results of the mechanical testing are shown in Table 5.

**Table 5 Results of the mechanical testing of IE 4 to IE 7 and CE 7 to CE 13**

| | **CE 7** | **CE 8** | **CE 9** | **IE 4** | **IE 5** | **CE 10** | **CE 11** | **CE 12** | **CE 13** | **IE 6** | **IE 7** |
|---|---|---|---|---|---|---|---|---|---|---|---|
| **Film thickness / micron µm** | 13 | 13 | 13 | 13 | 13 | 12 | 12 | 11 | 12 | 12 | 12 |

| **Tensile test machine direction** | | | | | | | | | | | |
|---|---|---|---|---|---|---|---|---|---|---|---|
| **E-Modulus / MPa** | 144 | 309 | 292 | 300 | 292 | 345 | 612 | 577 | 545 | 544 | 487 |
| **Max. tensile strength / MPa** | 30.1 | 32.2 | 31.6 | 31.5 | 32.1 | 34.9 | 43.2 | 33.6 | 33.3 | 32.7 | 38.2 |
| **Elongation at maximum tensile strength / %** | 481 | 251 | 269 | 336 | 328 | 189 | 173 | 153 | 185 | 312 | 326 |

| **Tensile test transversal direction** | | | | | | | | | | | |
|---|---|---|---|---|---|---|---|---|---|---|---|
| **E-Modulus / MPa** | 136 | 200 | 209 | 264 | 231 | 197 | 444 | 433 | 456 | 392 | 426 |
| **Max. tensile strength / MPa** | 23.3 | 25.2 | 26.2 | 20.8 | 22.0 | 32.1 | 35.7 | 23.2 | 25.4 | 23.6 | 31.8 |
| **Elongation at maximum tensile strength** / **%** | 552 | 509 | 534 | 469 | 473 | 508 | 404 | 389 | 410 | 395 | 512 |
| | | | | | | | | | | | |

| **Tear resistance / mN** | | | | | | | | | | | |
|---|---|---|---|---|---|---|---|---|---|---|---|
| **Machine direction** | 1798 | 1490 | 2355 | 798 | 972 | 1423 | 94 | 761 | 1316 | 974 | 1982 |
| **Transversal direction** | 2922 | 1656 | 1820 | 3199 | 3000 | 267 | 129 | 118 | 229 | 1364 | 1218 |

CE 7 is a blend made from a composition containing no PLA. It can be seen that the addition of common PLA in the comparative examples increases the E-modulus in both directions, decreases the elongation at maximum tensile strength in machine direction and decreases the tear resistance in transversal direction. The effects are even more pronounced at higher common PLA content.

In case a poly(meso-lactide) is added instead of a common PLA the decrease of the elongation at maximum tensile strength is clearly smaller. Additionally, the tear resistance in transversal direction is much higher in films containing poly(meso-lactide) than in films containing a common PLA. Furthermore, in all films containing poly(meso-lactide) and a desirably high content of 40 wt.-% TPS, the ratio of the tear resistance in transversal direction versus machine direction is larger than 1, whereas for films containing a common PLA it is usually smaller than or close to 1.

## Claims

1. Biodegradable polymer composition containing
a1) 2 to 50 wt.-%, based on the total weight of components a1) to e) of the polymer composition, of at least one poly(meso-lactide) having a number-average molecular weight of at least 20 000 g/mol, and a ratio of L-lactic units : D-lactic units of at least 20:80 up to 80:20;
a2) 0 to 48 wt.-%, based on the total weight of components a1) to e), of at least one polylactide a2) having a ratio of L-lactic units : D-lactic units above 65:35 or below 35:65 and wherein the sum of poly(meso-lactide) a1) and polylactide a2) is at maximum 50 wt.-%, based on the total weight of components a1) to e);
b) 20 to 98 wt.-%, based on the total weight of components a1) to e) of the polymer composition, of at least one biodegradable polyester selected from aliphatic polyesters, aliphatic-aromatic polyesters and copolymers and mixtures thereof;
c) 0 to 55 wt.-%, based on the total weight of components a1) to e) of the polymer composition, of at least one starch polymer;
d) 0 to 40 wt.-%, based on the total weight of components a1) to e) of the polymer composition, of at least one inorganic filler; and
e) 0 to 5 wt%, based on the total weight of components a1) to e) of the polymer composition, of at least one compound selected from cross-linking agents, lubricants, chain extenders, stabilizers, nucleating agents, slip and release agents, surfactants, waxes, antistatic agents, antifoggants, dyes, pigments, UV absorbers, UV stabilizers and other plastics additives; wherein the number-average molecular weight of the poly(meso-lactide) and the ratio of L-lactic units : D-lactic units were determined as indicated in the description.

2. The biodegradable polymer composition according to claim 1 wherein the ratio of L-lactic units : D-lactic units in the poly(meso-lactide) a1) is at least 35:65 up to 65:35.

3. The biodegradable polymer composition according to claim 1 or 2, wherein the poly(meso-lactide) a1) has an average block length of L-lactic units and of D-lactic units of at least 1.0 up to 2.0.

4. The biodegradable polymer composition according to any of claims 1 to 3 wherein the weight ratio of the poly(meso-lactide) a1) to the polylactide a2) is at least 1:3.

5. The biodegradable polymer composition according to any of claims 1 to 4 wherein the poly(meso-lactide) a1) a has a glass transition temperature of 38 to 46 °C measured by DSC at a relative humidity of 100 % and a heating rate of 20 °C/min.

6. The biodegradable polymer composition according to any of claims 1 to 5 wherein the polylactide a2) has an average block length of L-lactic units and/or of D-lactic units above 2.

7. The biodegradable polymer composition according to any of claims 1 to 6, wherein the poly(meso-lactide) a1) has an average block length of L-lactic units and D-lactic units of at least 1.1 and up to 1.75.

8. The biodegradable polymer composition according to any of claims 1 to 7, wherein the poly(meso-lactide) a1) has a number average molecular weight of from 30 000 g/mol up to 130 000 g/mol.

9. The biodegradable polymer composition according to any of claims 1 to 8, wherein the biodegradable polyester b) comprises at least one aliphatic-aromatic polyester derived from:
b-1) 20 bis 70 mol %, based on the total amount of components b-1) and b-2), of at least one aliphatic C₄-C₁₈ dicarboxylic acid or C₄-C₁₈ dicarboxylic acid derivative;
b-2) 80 bis 30 mol %, based on the total amount of components b-1) and b-2), of at least one aromatic dicarboxylic acid or aromatic dicarboxylic acid derivative;
b-3) 98 to 100 mol %, based on the total amount of b-1) and b-2), of an aliphatic C₂-C₁₀ diol;
b-4) 0 to 2 wt%, based on the total weight of components b-1), b-2) and b-3), of an at least trihydric alcohol; and
b-5) 0 to 2 wt%, based on the total weight of components b-1), b-2) and b-3), of a chain extender.

10. The biodegradable polymer composition according to claim 9, wherein the aliphatic C₄-C₁₈ dicarboxylic acid and its derivative b-1) are selected from succinic acid, adipic acid, azelaic acid, sebacic acid, 1,12-dodecanedioic acid, brassylic acid, their derivatives, and mixtures thereof; the aromatic acid and aromatic acid derivative b-2) are selected from terephthalic acid, 2,5-furandicarboxylic acid, their derivatives and mixtures thereof; and the diol b-3) is 1,4-butanediol.

11. The biodegradable polymer composition according to any of claims 1 to 8, wherein the biodegradable polyester b) comprises at least one aliphatic polyester derived from:
b-1) at least one aliphatic C₄-C₁₈ dicarboxylic acid or C₄-C₁₈ dicarboxylic acid derivative,
b-3) 98 to 100 mol %, based on the total amount of b-1), of at least one aliphatic C₂-C₁₀ diol;
b-4) 0 to 2 wt%, based on the total weight of components b-1) and b-3), of an at least trihydric alcohol; and
b-5) 0 to 2 wt%, based on the total weight of components b-1) and b-3), of a chain extender
or derived from:
b-6) at least one C₂-C₁₈ hydroxycarboxylic acid or C₂-C₁₈ hydroxycarboxylic acid derivative; and
b-4) 0 to 2 wt%, based on the total weight of component b-6), of an at least trihydric alcohol; and
b-5) 0 to 2 wt%, based on the total weight of component b-6), of a chain extender;
or a mixture thereof.

12. The biodegradable polymer composition according to claim 11, wherein the aliphatic C₄-C₁₈ dicarboxylic acid and its derivative b-1) are selected from succinic acid, adipic acid, azelaic acid, sebacic acid, 1,12-dodecanedioic acid, and brassylic acid, their derivatives, and mixtures thereof; and the diol b-3) is 1,4-butanediol and the C₂-C₁₈ hydroxycarboxylic acid and its derivative b-6) is selected from glycolic acid, hydroxypropionic acid, hydroxybutanoic acid, hydroxyvaleric acid, hydroxyhexanoic acid, and caprolactone.

13. The biodegradable polymer composition according to any of claims 1 to 12 containing at least one starch polymer c) selected from flour, native starch, modified starch, hydrolyzed starch, destructured starch, gelatinized starch, plasticized starch, thermoplastic starch, and mixtures thereof.

14. The biodegradable polymer composition according to any of claims 1 to 13 containing at least one inorganic filler selected from salts of alkaline earth metals, silicic acids and their salts, silica gel, silicates, silicon dioxide (quartz), bentonite, graphite, carbon black, iron oxide, kaolin, sodium carbonate, titanium dioxide, wollastonite, mica, bentonite, montmorillonites, and mineral fibers.

15. The biodegradable polymer composition according to any of claims 1 to 14, containing
a1) 2 to 30 wt.-%, based on the total weight of components a1) to e) of the polymer composition, of at least one poly(meso-lactide);
a2) 0 to 28 wt.-% based on the total weight of components a1) to e) of the polymer composition, of at least one polylactide a2), wherein the sum of the weight percentages of poly(meso-lactide) a1) and polylactide a2) is at maximum 30 wt.-% based on components a1) to e) of the polymer composition;
b) 40 to 96 wt.-%, based on the total weight of components a1) to e) of the polymer composition, of at least one biodegradable aliphatic or aliphatic-aromatic polyester;
c) 2 to 30 wt.-%, based on the total weight of components a1) to e) of the polymer composition, of at least one starch polymer;
d) 0 to 30 wt.-%, based on the total weight of components a1) to e) of the polymer composition, of at least one inorganic filler; and
e) 0 to 5 wt%, based on the total weight of components a1) to e) of the polymer composition, of at least one compound selected from cross-linking agents, lubricants, chain extenders, stabilizers, nucleating agents, slip and release agents, surfactants, waxes, antistatic agents, antifoggants, dyes, pigments, UV absorbers, UV stabilizers and other plastics additives.

16. The biodegradable polymer composition according to any of claims 1 to 14 containing
a1) 2 to 40 wt.-%, based on the total weight of components a1) to e) of the polymer composition, of at least one poly(meso-lactide);
a2) 0 to 38 wt.-% based on the total weight of components a1) to e) of the polymer composition, of at least one polylactide a2), wherein the sum of the weight percentages of poly(meso-lactide) a1) and polylactide a2) is at maximum 40 wt.-%, based on components a1) to e) of the polymer composition;
b) 20 to 73 wt.-%, based on the total weight of components a1) to e) of the polymer composition, of at least one biodegradable aliphatic or aliphatic-aromatic polyester;
c) 25 to 55 wt.-%, based on the total weight of components a1) to e) of the polymer composition, of at least one starch polymer;
d) 0 to 30 wt.-%, based on the total weight of components a1) to e) of the polymer composition, of at least one inorganic filler; and
e) 0 to 5 wt%, based on the total weight of components a1) to e) of the polymer composition, of at least one compound selected from cross-linking agents, lubricants, chain extenders, stabilizers, nucleating agents, slip and release agents, surfactants, waxes, antistatic agents, antifoggants, dyes, pigments, UV absorbers, UV stabilizers and other plastics additives.

17. The biodegradable polymer composition according to any of claims 1 to 14, wherein the polymer composition contains
a1) 2 to 20 wt.-%, based on the total weight of components a1) to e) of the polymer composition, of at least one poly(meso-lactide);
a2) 0 to 18 wt.-%, based on the total weight of components a1) to e) of the polymer composition, of at least one polylactide a2), wherein the sum of the weight percentages of poly(meso-lactide) a1) and polylactide a2) is at maximum 20 wt.-%, based on components a1) to e) of the polymer composition;
b) 55 to 98 wt.-%, based on the total weight of components a1) to e) of the polymer composition, of at least one biodegradable aliphatic or aliphatic-aromatic polyester;
c) 0 to 10 wt.-%, based on the total weight of components a1) to e) of polymer composition, of at least one starch polymer;
d) 0 to 10 wt.-%, based on the total weight of components a1) to e) of the polymer composition, of at least one inorganic filler; and
e) 0 to 5 wt%, based on the total weight of components a1) to e) of the polymer composition, of at least one compound selected from cross-linking agents, lubricants, chain extenders, stabilizers, nucleating agents, slip and release agents, surfactants, waxes, antistatic agents, antifoggants, dyes, pigments, UV absorbers, UV stabilizers and other plastics additives.

18. A film comprising the biodegradable polymer composition according to any of claims 1 to 17.

19. Packaging, carrier bag, fruit or vegetable bag, waste bag or mulch film comprising a film according to claim 18.

## Patentansprüche

1. Biologisch abbaubare Polymerzusammensetzung enthaltend
a1) 2 bis 50 Gew.-%, bezogen auf das Gesamtgewicht der Komponenten a1) bis e) der Polymerzusammensetzung, an wenigstens einem Poly(meso-lactid) mit einem anzahlgemittelten Molekulargewicht von wenigstens 20 000 g/mol und einem Verhältnis von L-Milchsäureeinheiten: D-Milchsäureeinheiten von wenigstens 20:80 bis zu 80:20;
a2) 0 bis 48 Gew.-%, bezogen auf das Gesamtgewicht der Komponenten a1) bis e), an wenigstens einem Polylactid a2) mit einem Verhältnis von L-Milchsäureeinheiten: D-Milchsäureeinheiten von über 65:35 oder unter35:65, und wobei die Summe von Poly(meso-lactid) a1) und Polylactid a2) höchstens 50 Gew.-%, bezogen auf das Gesamtgewicht der Komponenten a1) bis e), beträgt;
b) 20 bis 98 Gew.-%, bezogen auf das Gesamtgewicht der Komponenten a1) bis e) der Polymerzusammensetzung, an wenigstens einem biologisch abbaubaren Polyester ausgewählt aus aliphatischen Polyestern, aliphatisch-aromatischen Polyestern und Copolymeren und Gemischen davon;
c) 0 bis 55 Gew.-%, bezogen auf das Gesamtgewicht der Komponenten a1) bis e) der Polymerzusammensetzung, an wenigstens einem Stärkepolymer;
d) 0 bis 40 Gew.-%, bezogen auf das Gesamtgewicht der Komponenten a1) bis e) der Polymerzusammensetzung, an wenigstens einem anorganischen Füllstoff; und
e) 0 bis 5 Gew.-%, bezogen auf das Gesamtgewicht der Komponenten a1) bis e) der Polymerzusammensetzung, an wenigstens einer Verbindung ausgewählt aus Vernetzern, Gleitmitteln, Kettenverlängerern, Stabilisatoren, Keimbildnern, Gleit- und Trennmitteln, Tensiden, Wachsen, antistatischen Mitteln, Antibeschlagmitteln, Farbstoffen, Pigmenten, UV-Absorbern, UV-Stabilisatoren und andere Kunststoffzusätzen; wobei das anzahlgemittelte Molekulargewicht des Poly(meso-lactids) und das Verhältnis von L-Milchsäureeinheiten: D-Milchsäureeinheiten wie in der Beschreibung beschrieben bestimmt wurden.

2. Biologisch abbaubare Polymerzusammensetzung nach Anspruch 1, wobei das Verhältnis von L-Milchsäureeinheiten: D-Milchsäureeinheiten in dem Poly(meso-lactid) a1) wenigstens 35:65 bis zu 65:35 beträgt.

3. Biologisch abbaubare Polymerzusammensetzung nach Anspruch 1 oder 2, wobei das Poly(meso-lactid) a1) eine mittlere Blocklänge von L-Milchsäureeinheiten und von D-Milchsäureeinheiten von wenigstens 1,0 bis zu 2,0 aufweist.

4. Biologisch abbaubare Polymerzusammensetzung nach einem der Ansprüche 1 bis 3, wobei das Gewichtsverhältnis des Poly(meso-lactids) a1) zu dem Polylactid a2) wenigstens 1:3 beträgt.

5. Biologisch abbaubare Polymerzusammensetzung nach einem der Ansprüche 1 bis 4, wobei das Poly(meso-lactid) a1) eine Glasübergangstemperatur von 38 bis 46 °C, gemessen durch DSC bei einer relativen Feuchtigkeit von 100 % und einer Heizrate von 20 °C/min, aufweist.

6. Biologisch abbaubare Polymerzusammensetzung nach einem der Ansprüche 1 bis 5, wobei das Polylactid a2) eine mittlere Blocklänge von L-Milchsäureeinheiten und/oder von D-Milchsäureeinheiten von größer als 2 aufweist.

7. Biologisch abbaubare Polymerzusammensetzung nach einem der Ansprüche 1 bis 6, wobei das Poly(meso-lactid) a1) eine mittlere Blocklänge von L-Milchsäureeinheiten und D-Milchsäureeinheiten von wenigstens 1,1 und bis zu 1,75 aufweist.

8. Biologisch abbaubare Polymerzusammensetzung nach einem der Ansprüche 1 bis 7, wobei das Poly(meso-lactid) a1) ein anzahlgemitteltes Molekulargewicht von 30 000 g/mol bis 130 000 g/mol aufweist.

9. Biologisch abbaubare Polymerzusammensetzung nach einem der Ansprüche 1 bis 8, wobei der biologisch abbaubare Polyester b) wenigstens einen aliphatisch-aromatischen Polyester umfasst, abgeleitet von:
b-1) 20 bis 70 mol-%, bezogen auf die Gesamtmenge der Komponenten b-1) und b-2), an wenigstens einer aliphatischen C₄-C₁₈-Dicarbonsäure oder einem C₄-C₁₈-Dicarbonsäurederivat;
b-2) 80 bis 30 mol-%, bezogen auf die Gesamtmenge der Komponenten b-1) und b-2), an wenigstens einer aromatischen Dicarbonsäure oder einem aromatischen Dicarbonsäurederivat;
b-3) 98 bis 100 mol-%, bezogen auf die Gesamtmenge von b-1) und b-2), an einem aliphatischen C₂-C₁₀-Diol;
b-4) 0 bis 2 Gew.-%, bezogen auf das Gesamtgewicht der Komponenten b-1), b-2) und b-3), an einem wenigstens dreiwertigen Alkohol; und
b-5) 0 bis 2 Gew.-%, bezogen auf das Gesamtgewicht der Komponenten b-1), b-2) und b-3), an einem Kettenverlängerer.

10. Biologisch abbaubare Polymerzusammensetzung nach Anspruch 9, wobei die aliphatische C₄-C₁₈-Dicarbonsäure und ihr Derivat b-1) ausgewählt sind aus Bernsteinsäure, Adipinsäure, Azelainsäure, Sebacinsäure, 1,12-Dodecandisäure, Brassylsäure, ihren Derivaten und Gemischen davon; die aromatische Säure und das aromatische Säurederivat b-2) ausgewählt sind aus Terephthalsäure, 2,5-Furandicarbonsäure, ihren Derivaten und Gemischen davon; und das Diol b-3) 1,4-Butandiol ist.

11. Biologisch abbaubare Polymerzusammensetzung nach einem der Ansprüche 1 bis 8, wobei der biologisch abbaubare Polyester b) wenigstens einen aliphatischen Polyester umfasst, abgeleitet von:
b-1) wenigstens einer aliphatischen C₄-C₁₈-Dicarbonsäure oder einem C₄-C₁₈-Dicarbonsäurederivat,
b-3) 98 bis 100 mol-%, bezogen auf die Gesamtmenge von b-1), an wenigstens einem aliphatischen C₂-C₁₀-Diol;
b-4) 0 bis 2 Gew.-%, bezogen auf das Gesamtgewicht der Komponenten b-1) und b-3), an einem wenigstens dreiwertigen Alkohol; und
b-5) 0 bis 2 Gew.-%, bezogen auf das Gesamtgewicht der Komponenten b-1) und b-3), an einem Kettenverlängerer oder abgeleitet von:
b-6) wenigstens einer C₂-C₁₈-Hydroxycarbonsäure oder einem C₂-C₁₈-Hydroxycarbonsäurederivat; und
b-4) 0 bis 2 Gew.-%, bezogen auf das Gesamtgewicht der Komponente b-6), an einem wenigstens dreiwertigen Alkohol; und
b-5) 0 bis 2 Gew.-%, bezogen auf das Gesamtgewicht der Komponente b-6), an einem Kettenverlängerer; oder einem Gemisch davon.

12. Biologisch abbaubare Polymerzusammensetzung nach Anspruch 11, wobei die aliphatische C₄-C₁₈-Dicarbonsäure und ihr Derivat b-1) ausgewählt sind aus Bernsteinsäure, Adipinsäure, Azelainsäure, Sebacinsäure, 1,12-Dodecandisäure und Brassylsäure, ihren Derivaten und Gemischen davon; und das Diol b-3) 1,4-Butandiol ist und die C₂-C₁₈-Hydroxycarbonsäure und ihr Derivat b-6) ausgewählt sind aus Glycolsäure, Hydroxypropionsäure, Hydroxybutansäure, Hydroxyvaleriansäure, Hydroxyhexansäure und Caprolacton.

13. Biologisch abbaubare Polymerzusammensetzung nach einem der Ansprüche 1 bis 12, enthaltend wenigstens ein Stärkepolymer c) ausgewählt aus Mehl, nativer Stärke, modifizierter Stärke, hydrolysierter Stärke, destrukturierter Stärke, gelatinierter Stärke, weichgemachter Stärke, thermoplastischer Stärke und Gemischen davon.

14. Biologisch abbaubare Polymerzusammensetzung nach einem der Ansprüche 1 bis 13, enthaltend wenigstens einen anorganischen Füllstoff ausgewählt aus Salzen von Erdalkalimetallen, Kieselsäure und ihren Salzen, Kieselgel, Silicaten, Siliciumdioxid (Quarz), Bentonit, Graphit, Ruß, Eisenoxid, Kaolin, Natriumcarbonat, Titandioxid, Wollastonit, Glimmer, Bentonit, Montmorilloniten und Mineralfasern.

15. Biologisch abbaubare Polymerzusammensetzung nach einem der Ansprüche 1 bis 14, enthaltend a1) 2 bis 30 Gew.-%, bezogen auf das Gesamtgewicht der Komponenten a1) bis e) der Polymerzusammensetzung, an wenigstens einem Poly(meso-lactid);
a2) 0 bis 28 Gew.-%, bezogen auf das Gesamtgewicht der Komponenten a1) bis e) der Polymerzusammensetzung, an wenigstens einem Polylactid a2), wobei die Summe der Gewichtsprozentwerte von Poly(meso-lactid) a1) und Polylactid a2) höchstens 30 Gew.-%, bezogen auf die Komponenten a1) bis e) der Polymerzusammensetzung, beträgt;
b) 40 bis 96 Gew.-%, bezogen auf das Gesamtgewicht der Komponenten a1) bis e) der Polymerzusammensetzung, an wenigstens einem biologisch abbaubaren aliphatischen oder aliphatisch-aromatischen Polyester;
c) 2 bis 30 Gew.-%, bezogen auf das Gesamtgewicht der Komponenten a1) bis e) der Polymerzusammensetzung, an wenigstens einem Stärkepolymer;
d) 0 bis 30 Gew.-%, bezogen auf das Gesamtgewicht der Komponenten a1) bis e) der Polymerzusammensetzung, an wenigstens einem anorganischen Füllstoff; und
e) 0 bis 5 **Gew.-%,** bezogen auf das Gesamtgewicht der Komponenten a1) bis e) der Polymerzusammensetzung, an wenigstens einer Verbindung ausgewählt aus Vernetzern, Gleitmitteln, Kettenverlängerern, Stabilisatoren, Keimbildnern, Gleit- und Trennmitteln, Tensiden, Wachsen, antistatischen Mitteln, Antibeschlagmitteln, Farbstoffen, Pigmenten, UV-Absorbern, UV-Stabilisatoren und anderen Kunststoffzusätzen.

16. Biologisch abbaubare Polymerzusammensetzung nach einem der Ansprüche 1 bis 14, enthaltend
a1) 2 bis 40 **Gew.-%,** bezogen auf das Gesamtgewicht der Komponenten a1) bis e) der Polymerzusammensetzung, an wenigstens einem Poly(meso-lactid);
a2) 0 bis 38 **Gew.-%,** bezogen auf das Gesamtgewicht der Komponenten a1) bis e) der Polymerzusammensetzung, an wenigstens einem Polylactid a2), wobei die Summe der Gewichtsprozentwerte von Poly(meso-lactid) a1) und Polylactid a2) höchstens 40 **Gew.-%,** bezogen auf die Komponenten a1) bis e) der Polymerzusammensetzung, beträgt;
b) 20 bis 73 Gew.-%, bezogen auf das Gesamtgewicht der Komponenten a1) bis e) der Polymerzusammensetzung, an wenigstens einem biologisch abbaubaren aliphatischen oder aliphatisch-aromatischen Polyester;
c) 25 bis 55 Gew.-%, bezogen auf das Gesamtgewicht der Komponenten a1) bis e) der Polymerzusammensetzung, an wenigstens einem Stärkepolymer;
d) 0 bis 30 Gew.-%, bezogen auf das Gesamtgewicht der Komponenten a1) bis e) der Polymerzusammensetzung, an wenigstens einem anorganischen Füllstoff; und
e) 0 bis 5 Gew.-%, bezogen auf das Gesamtgewicht der Komponenten a1) bis e) der Polymerzusammensetzung, an wenigstens einer Verbindung ausgewählt aus Vernetzern, Gleitmitteln, Kettenverlängerern, Stabilisatoren, Keimbildnern, Gleit- und Trennmitteln, Tensiden, Wachsen, antistatischen Mitteln, Antibeschlagmitteln, Farbstoffen, Pigmenten, UV-Absorbern, UV-Stabilisatoren und anderen Kunststoffzusätzen.

17. Biologisch abbaubare Polymerzusammensetzung nach einem der Ansprüche 1 bis 14, wobei die Polymerzusammensetzung enthält
a1) 2 bis 20 Gew.-%, bezogen auf das Gesamtgewicht der Komponenten a1) bis e) der Polymerzusammensetzung, an wenigstens einem Poly(meso-lactid);
a2) 0 bis 18 Gew.-%, bezogen auf das Gesamtgewicht der Komponenten a1) bis e) der Polymerzusammensetzung, an wenigstens einem Polylactid a2), wobei die Summe der Gewichtsprozentwerte von Poly(meso-lactid) a1) und Polylactid a2) höchstens 20 Gew.-%, bezogen auf die Komponenten a1) bis e) der Polymerzusammensetzung, beträgt;
b) 55 bis 98 Gew.-%, bezogen auf das Gesamtgewicht der Komponenten a1) bis e) der Polymerzusammensetzung, an wenigstens einem biologisch abbaubaren aliphatischen oder aliphatisch-aromatischen Polyester;
c) 0 bis 10 Gew.-%, bezogen auf das Gesamtgewicht der Komponenten a1) bis e) der Polymerzusammensetzung, an wenigstens einem Stärkepolymer;
d) 0 bis 10 Gew.-%, bezogen auf das Gesamtgewicht der Komponenten a1) bis e) der Polymerzusammensetzung, an wenigstens einem anorganischen Füllstoff; und
e) 0 bis 5 Gew.-%, bezogen auf das Gesamtgewicht der Komponenten a1) bis e) der Polymerzusammensetzung, an wenigstens einer Verbindung ausgewählt aus Vernetzern, Gleitmitteln, Kettenverlängerern, Stabilisatoren, Keimbildnern, Gleit- und Trennmitteln, Tensiden, Wachsen, antistatischen Mitteln, Antibeschlagmitteln, Farbstoffen, Pigmenten, UV-Absorbern, UV-Stabilisatoren und anderen Kunststoffzusätzen.

18. Folie umfassend die biologisch abbaubare Polymerzusammensetzung nach einem der Ansprüche 1 bis 17.

19. Verpackung, Tragebeutel, Obst- oder Gemüsebeutel, Abfallbeutel oder Mulchfolie umfassend eine Folie nach Anspruch 18.

## Revendications

1. Composition de polymère biodégradable contenant
a1) 2 à 50 % en poids, par rapport au poids total des composants a1) à e) de la composition de polymère, d'au moins un poly(méso-lactide) ayant un poids moléculaire moyen en nombre d'au moins 20 000 g/mole, et un rapport de motifs L-lactiques : motifs D-lactiques d'au moins 20:80 jusqu'à 80:20 ;
a2) 0 à 48 % en poids, par rapport au poids total des composants a1) à e), d'au moins un polylactide a2) ayant un rapport de motifs L-lactiques : motifs D-lactiques supérieur à 65:35 ou inférieur à 35:65 et dans laquelle la somme du poly(méso-lactide) a1) et du polylactide a2) est au maximum de 50 % en poids, par rapport au poids total des composants a1) à e) ;
b) 20 à 98 % en poids, par rapport au poids total des composants a1) à e) de la composition de polymère, d'au moins un polyester biodégradable choisi parmi les polyesters aliphatiques, les polyesters aliphatique-aromatiques et leurs copolymères et leurs mélanges ;
c) 0 à 55 % en poids, par rapport au poids total des composants a1) à e) de la composition de polymère, d'au moins un polymère d'amidon ;
d) 0 à 40 % en poids, par rapport au poids total des composants a1) à e) de la composition de polymère, d'au moins une charge inorganique ; et
e) 0 à 5 % en poids, par rapport au poids total des composants a1) à e) de la composition de polymère, d'au moins un composé choisi parmi les agents de réticulation, les lubrifiants, les extenseurs de chaîne, les stabilisants, les agents de nucléation, les agents de glissement et de libération, les tensioactifs, les cires, les agents antistatiques, les agents anti-brouillard, les colorants, les pigments, les absorbeurs d'UV, les stabilisants aux UV et d'autres additifs de matières plastiques ; dans laquelle le poids moléculaire moyen en nombre du poly(méso-lactide) et le rapport des motifs L-lactiques : motifs D-lactiques ont été déterminés comme indiqué dans la description.

2. Composition de polymère biodégradable selon la revendication 1, dans laquelle le rapport des motifs L-lactiques : motifs D-lactiques dans le poly(méso-lactide) a1) est d'au moins 35:65 jusqu'à 65:35.

3. Composition de polymère biodégradable selon la revendication 1 ou 2, dans laquelle le poly(méso-lactide) a1) a une longueur de bloc moyenne de motifs L-lactiques et de motifs D-lactiques d'au moins 1,0 jusqu'à 2,0.

4. Composition de polymère biodégradable selon l'une quelconque des revendications 1 à 3, dans laquelle le rapport pondéral du poly(méso-lactide) a1) sur le polylactide a2) est d'au moins 1:3.

5. Composition de polymère biodégradable selon l'une quelconque des revendications 1 à 4, dans laquelle le poly(méso-lactide) a1) a a une température de transition vitreuse de 38 à 46 °C mesurée par DSC à une humidité relative de 100 % et une vitesse de chauffage de 20 °C/min.

6. Composition de polymère biodégradable selon l'une quelconque des revendications 1 à 5, dans laquelle le polylactide a2) a une longueur de bloc moyenne de motifs L-lactiques et/ou de motifs D-lactiques supérieure à 2.

7. Composition de polymère biodégradable selon l'une quelconque des revendications 1 à 6, dans laquelle le poly(méso-lactide) a1) a une longueur de bloc moyenne de motifs L-lactiques et de motifs D-lactiques d'au moins 1,1 et jusqu'à 1,75.

8. Composition de polymère biodégradable selon l'une quelconque des revendications 1 à 7, dans laquelle le poly(méso-lactide) a1) a un poids moléculaire moyen en nombre de 30 000 g/mole à 130 000 g/mole.

9. Composition de polymère biodégradable selon l'une quelconque des revendications 1 à 8, dans laquelle le polyester biodégradable b) comprend au moins un polyester aliphatique-aromatique dérivé de :
b-1) 20 à 70 % en moles, par rapport à la quantité totale des composants b-1) et b-2), d'au moins un acide dicarboxylique aliphatique en C₄-C₁₈ ou dérivé d'acide dicarboxylique en C₄-C₁₈ ;
b-2) 80 à 30 % en moles, par rapport à la quantité totale des composants b-1) et b-2), d'au moins un acide dicarboxylique aromatique ou dérivé d'acide dicarboxylique aromatique ;
b-3) 98 à 100 % en moles, par rapport à la quantité totale de b-1) et b-2), d'un diol aliphatique en C₂-C₁₀ ;
b-4) 0 à 2 % en poids, par rapport au poids total des composants b-1), b-2) et b-3), d'un alcool au moins trihydrique ; et
b-5) 0 à 2 % en poids, par rapport au poids total des composants b-1), b-2) et b-3), d'un extenseur de chaîne.

10. Composition de polymère biodégradable selon la revendication 9, dans laquelle l'acide dicarboxylique aliphatique en C₄-C₁₈ et son dérivé b-1) sont choisis parmi l'acide succinique, l'acide adipique, l'acide azélaïque, l'acide sébacique, l'acide 1,12-dodécanedioïque, l'acide brassylique, leurs dérivés et leurs mélanges ; l'acide aromatique et le dérivé d'acide aromatique b-2) sont choisis parmi l'acide téréphtalique, l'acide 2,5-furanedicarboxylique, leurs dérivés et leurs mélanges ; et le diol b-3) est le 1,4-butanediol.

11. Composition de polymère biodégradable selon l'une quelconque des revendications 1 à 8, dans laquelle le polyester biodégradable b) comprend au moins un polyester aliphatique dérivé de :
b-1) au moins un acide dicarboxylique aliphatique en C₄-C₁₈ ou dérivé d'acide dicarboxylique en C₄-C₁₈,
b-3) 98 à 100 % en moles, par rapport à la quantité totale de b-1), d'au moins un diol aliphatique en C₂-C₁₀ ;
b-4) 0 à 2 % en poids, par rapport au poids total des composants b-1) et b-3), d'un alcool au moins trihydrique ; et
b-5) 0 à 2 % en poids, par rapport au poids total des composants b-1) et b-3), d'un extenseur de chaîne ou dérivé de :
b-6) au moins un acide hydroxycarboxylique en C₂-C₁₈ ou dérivé d'acide hydroxycarboxylique en C₂-C₁₈ ; et
b-4) 0 à 2 % en poids, par rapport au poids total du composant b-6), d'un alcool au moins trihydrique ; et
b-5) 0 à 2 % en poids, par rapport au poids total du composant b-6), d'un extenseur de chaîne ; ou d'un mélange de ceux-ci.

12. Composition de polymère biodégradable selon la revendication 11, dans laquelle l'acide dicarboxylique aliphatique en C₄-C₁₈ et son dérivé b-1) sont choisis parmi l'acide succinique, l'acide adipique, l'acide azélaïque, l'acide sébacique, l'acide 1,12-dodécanedioïque, et l'acide brassylique, leurs dérivés et leurs mélanges ; et le diol b-3) est le 1,4-butanediol et l'acide hydroxycarboxylique en C₂-C₁₈ et son dérivé b-6) est choisi parmi l'acide glycolique, l'acide hydroxypropionique, l'acide hydroxybutanoïque, l'acide hydroxyvalérique, l'acide hydroxyhexanoïque, et la caprolactone.

13. Composition de polymère biodégradable selon l'une quelconque des revendications 1 à 12, contenant au moins un polymère d'amidon c) choisi parmi la farine, l'amidon natif, l'amidon modifié, l'amidon hydrolysé, l'amidon déstructuré, l'amidon gélatinisé, l'amidon plastifié, l'amidon thermoplastique et leurs mélanges.

14. Composition de polymère biodégradable selon l'une quelconque des revendications 1 à 13, contenant au moins une charge inorganique choisie parmi les sels de métaux alcalino-terreux, les acides siliciques et leurs sels, le gel de silice, les silicates, le dioxyde de silicium (quartz), la bentonite, le graphite, le noir de carbone, l'oxyde de fer, le kaolin, le carbonate de sodium, le dioxyde de titane, la wollastonite, le mica, la bentonite, les montmorillonites et les fibres minérales.

15. Composition de polymère biodégradable selon l'une quelconque des revendications 1 à 14, contenant
a1) 2 à 30 % en poids, par rapport au poids total des composants a1) à e) de la composition de polymère, d'au moins un poly(méso-lactide) ;
a2) de 0 à 28 % en poids par rapport au poids total des composants a1) à e) de la composition de polymère, d'au moins un polylactide a2), la somme des pourcentages en poids de poly(méso-lactide) a1) et de polylactide a2) étant au maximum de 30 % en poids, par rapport aux composants a1) à e) de la composition de polymère ;
b) 40 à 96 % en poids, par rapport au poids total des composants a1) à e) de la composition de polymère, d'au moins un polyester aliphatique ou aliphatique-aromatique biodégradable ;
c) 2 à 30 % en poids, par rapport au poids total des composants a1) à e) de la composition de polymère, d'au moins un polymère d'amidon ;
d) 0 à 30 % en poids, par rapport au poids total des composants a1) à e) de la composition de polymère, d'au moins une charge inorganique ; et
e) 0 à 5 % en poids, par rapport au poids total des composants a1) à e) de la composition de polymère, d'au moins un composé choisi parmi les agents de réticulation, les lubrifiants, les extenseurs de chaîne, les stabilisants, les agents de nucléation, les agents de glissement et de libération, les tensioactifs, les cires, les agents antistatiques, les agents anti-brouillard, les colorants, les pigments, les absorbeurs d'UV, les stabilisants aux UV et d'autres additifs de matières plastiques.

16. Composition de polymère biodégradable selon l'une quelconque des revendications 1 à 14 contenant
a1) 2 à 40 % en poids, par rapport au poids total des composants a1) à e) de la composition de polymère, d'au moins un poly(méso-lactide) ;
a2) 0 à 38 % en poids, par rapport au poids total des composants a1) à e) de la composition de polymère, d'au moins un polylactide a2), la somme des pourcentages en poids de poly(méso-lactide) a1) et de polylactide a2) étant au maximum de 40 % en poids, par rapport aux composants a1) à e) de la composition de polymère ;
b) 20 à 73 % en poids, par rapport au poids total des composants a1) à e) de la composition de polymère, d'au moins un polyester aliphatique ou aliphatique-aromatique biodégradable ;
c) 25 à 55 % en poids, par rapport au poids total des composants a1) à e) de la composition de polymère, d'au moins un polymère d'amidon ;
d) 0 à 30 % en poids, par rapport au poids total des composants a1) à e) de la composition de polymère, d'au moins une charge inorganique ; et
e) 0 à 5 % en poids, par rapport au poids total des composants a1) à e) de la composition de polymère, d'au moins un composé choisi parmi les agents de réticulation, les lubrifiants, les extenseurs de chaîne, les stabilisants, les agents de nucléation, les agents de glissement et de libération, les tensioactifs, les cires, les agents antistatiques, les agents anti-brouillard, les colorants, les pigments, les absorbeurs d'UV, les stabilisants aux UV et d'autres additifs de matières plastiques.

17. Composition de polymère biodégradable selon l'une quelconque des revendications 1 à 14, dans laquelle la composition de polymère contient
a1) 2 à 20 % en poids, par rapport au poids total des composants a1) à e) de la composition de polymère, d'au moins un poly(méso-lactide) ;
a2) 0 à 18 % en poids, par rapport au poids total des composants a1) à e) de la composition de polymère, d'au moins un polylactide a2), la somme des pourcentages en poids de poly(méso-lactide) a1) et de polylactide a2) étant au maximum de 20 % en poids, par rapport aux composants a1) à e) de la composition de polymère ;
b) 55 à 98 % en poids, par rapport au poids total des composants a1) à e) de la composition de polymère, d'au moins un polyester aliphatique ou aliphatique-aromatique biodégradable ;
c) 0 à 10 % en poids, par rapport au poids total des composants a1) à e) de la composition de polymère, d'au moins un polymère d'amidon ;
d) 0 à 10 % en poids, par rapport au poids total des composants a1) à e) de la composition de polymère, d'au moins une charge inorganique ; et
e) 0 à 5 % en poids, par rapport au poids total des composants a1) à e) de la composition de polymère, d'au moins un composé choisi parmi les agents de réticulation, les lubrifiants, les extenseurs de chaîne, les stabilisants, les agents de nucléation, les agents de glissement et de libération, les tensioactifs, les cires, les agents antistatiques, les agents anti-brouillard, les colorants, les pigments, les absorbeurs d'UV, les stabilisants aux UV et d'autres additifs de matières plastiques.

18. Film comprenant la composition de polymère biodégradable selon l'une quelconque des revendication 1 à 17.

19. Emballage, sac de transport, sac à fruits ou légumes, sac à déchets ou film de paillage comprenant un film selon la revendication 18.
